# EUROPEAN PATENT APPLICATION

(11) **EP 4 016 434 A1**
(43) Date of publication of application: **22.06.2022**
(21) Application number: 20851589.0
(22) Date of filing: 15.06.2020
(51) Int. Cl.: G06Q 30/06, G06Q 30/02, G06F 3/01, G06K 9/00

(54) **VIRTUAL FITTING PROVISION DEVICE AND PROVISION METHOD THEREFOR**

(30) Priority: 12.08.2019 US 201962885796 P
(71) Applicant: LG Electronics Inc., SEOUL 07336 (KR)
(72) Inventor: LEE, Haein, Seoul 06772 (KR); JUNG, Jiyun, Seoul 06772 (KR)
(74) Representative: Katérle, Axel
(86) International application number: PCT/KR2020/007694
(87) International publication number: WO 2021/029529

(57) **Abstract**

The present invention relates to a virtual fitting provision device and a provision method therefor. A virtual fitting provision device according to the present invention comprises: a touch screen; a camera for receiving an image of a subject at the front; and a control unit for activating a virtual fitting mode when a hand gesture input of the subject is sensed through the image or a touch gesture input of the subject is sensed on the touch screen, inquiring about the gender and at least one preferred style of the subject, searching for at least one fashion item related to the inquired gender and preferred style, the fashion item including at least one from among at least one piece of clothing, shoes and an accessory, measuring the body size of the subject, scanning the face of the subject, creating a virtual avatar based on the gender, the body size and the face, and displaying, on the touch screen, a virtual fitting screen including the avatar on which the retrieved fashion item is worn.

## Description

### TECHNICAL FIELD

The present disclosure relates to an apparatus for providing a virtual fitting and method therefor, suitable for providing a virtual fitting service to a user located in front.

### BACKGROUND ART

Virtual Reality (VR) technology provides an object, a background and the like of a real world only as a Computer Graphic (CG) image, Augmented Reality (AR) technology provides a virtually-rendered CG image on a real thing image together, and Mixed Reality (MR) technology is a computer graphic technology that provides virtual objects mixed and combined in a real world. All of the VR, AR, MR, etc. described above are simply referred to as Extended Reality (XR) technology.

The above-described XR technology is also applied to a virtual fitting providing device such as a smart mirror, and the virtual fitting providing device captures a user in front using its own camera and provides a virtual fitting service using a virtual avatar corresponding to the captured image of the user.

However, a virtual fitting service of the related art simply shows a state of preset virtual clothes worn on a virtual avatar, and fails to provide a virtual avatar based on a gender, a preferred fitting style, a detailed body size and the like of a user.

### DISCLOSURE OF THE INVENTION

### TECHNICAL TASK

The present disclosure is directed to solve the above problem and other problems, and one technical task of the present disclosure is to provide an apparatus for providing a virtual fitting and method therefor that provide a virtual fitting service in which a user's preferred fitting style is reflected by a virtual avatar based on user's gender, body size and face.

Technical tasks obtainable from the present disclosure are non-limited by the above-mentioned technical tasks. And, other unmentioned technical tasks can be clearly understood from the following description by those having ordinary skill in the technical field to which the present disclosure pertains.

### TECHNICAL SOLUTIONS

In one technical aspect of the present disclosure, provided is an apparatus for providing a virtual fitting, the apparatus including a touchscreen, a camera receiving an image of a subject in front, and a controller configured to activate a virtual fitting mode if detecting an input of a hand gesture of the subject or an input of a touch gesture of the subject on the touchscreen, inquire a gender of the subject and at least one preferred style, search for at least one fashion item related to the inquired gender and the preferred style, the fashion item including at least one of at least one clothes, shoes and accessory, measure a body size of the subject, scan a face of the subject, create a virtual avatar based on the gender, the body size and the face, and display a virtual fitting screen including the avatar wearing the found fashion item on the touchscreen.

In another technical aspect of the present disclosure, provided is a method for providing a virtual fitting in a virtual fitting providing device, the method including receiving an image of a subject in front through a camera, activating a virtual fitting mode if detecting an input of a hand gesture of the subject or an input of a touch gesture of the subject on the touchscreen, inquiring a gender of the subject and at least one preferred style, searching for at least one fashion item related to the inquired gender and the preferred style, the fashion item including at least one of at least one clothes, shoes and accessory, measuring a body size of the subject, scanning a face of the subject, creating a virtual avatar based on the gender, the body size and the face, and displaying a virtual fitting screen including the avatar wearing the found fashion item on the touchscreen.

### ADVANTAGEOUS EFFECTS

Accordingly, effects of the present disclosure are described as follows.

According to at least one of embodiments of the present disclosure, a virtual avatar based on a user's gender, preferred fitting style, detailed body size and the like is provided, thereby providing a more realistic virtual fitting service to the user.

Further scope of applicability of the present invention will become apparent from the detailed description given hereinafter. However, it should be understood that the detailed description and specific examples, while indicating preferred embodiments of the invention, are given by illustration only, since various changes and modifications within the spirit and scope of the invention will become apparent to those skilled in the art from this detailed description.

### DESCRIPTION OF DRAWINGS

FIG. 1 is a block diagram to describe a mobile terminal related to the present disclosure.
FIG. 2 is a conceptual diagram illustrating one example of a virtual fitting providing device related to the present disclosure.
FIG. 3 is a flowchart illustrating a process for providing a virtual fitting according to the present disclosure.
FIG. 4 is a flowchart illustrating an operating process of a simple fitting mode of a virtual fitting service according to the present disclosure.
FIG. 5 is a flowchart illustrating an operating process of a detailed fitting mode of a virtual fitting service according to the present disclosure.
FIG. 6 is a diagram to describe an intro screen of a virtual fitting service according to the present disclosure.
FIG. 7 is a diagram to describe a process for switching from a simple fitting mode to a detailed fitting mode according to the present disclosure.
FIGs. 8 to 10 are diagrams to describe a hand gesture tutorial screen in a simple fitting mode according to the present disclosure.
FIG. 11 is a diagram to describe a process for setting a gender of a subject in a simple fitting mode according to the present disclosure.
FIG. 12 is a diagram to describe a process for setting a gender of a subject in a detailed fitting mode according to the present disclosure.
FIG. 13 is a diagram to describe a process for setting a preferred fitting style in a simple fitting mode according to the present disclosure.
FIG. 14 is a diagram to describe a process for setting a preferred fitting style in a detailed fitting mode according to the present disclosure.
FIG. 15 and FIG. 16 are diagrams to describe a process for measuring a subject body size in a simple/detailed fitting mode according to the present disclosure.
FIG. 17 is a diagram to describe a process for scanning a subject face in a simple/detailed fitting mode according to the present disclosure.
FIG. 18 is a diagram illustrating a subject's body size report screen provided in a simple fitting mode according to the present disclosure.
FIG. 19 is a diagram illustrating a subject's body size report screen provided in a detailed fitting mode according to the present disclosure.
FIG. 20 is a diagram illustrating a look book screen including a virtual avatar in a simple fitting mode according to the present disclosure.
FIG. 21 is a diagram illustrating pages of virtual avatars wearing two or more fashion items in a simple fitting mode according to the present disclosure.
FIG. 22 is a diagram illustrating a process for transmitting information on a virtual avatar in a look book screen to an external mobile terminal in a simple/detailed fitting mode according to the present disclosure.
FIG. 23 is a diagram illustrating a process for making a payment for a fashion item worn on a virtual avatar in a simple fitting mode according to the present disclosure.
FIG. 24 is a diagram illustrating a look book screen including a virtual avatar in a detailed fitting mode according to the present disclosure.
FIG. 25 is a diagram illustrating a process for controlling a look book screen via a touch gesture in a detailed fitting mode according to the present disclosure.
FIGs. 26 to 29 are diagrams illustrating a process for changing and purchasing a fashion item worn on a virtual avatar in a detailed fitting mode according to the present disclosure.
FIG. 30 and FIG. 31 are diagrams illustrating a process for editing a virtual avatar in a detailed fitting mode according to the present disclosure.
FIG. 32 and FIG. 33 are diagrams illustrating a process for providing a fit map function in a detailed fitting mode according to the present disclosure.
FIG. 34 is a diagram illustrating a process for providing a My Closet function in a detailed fitting mode according to the present disclosure.
FIG. 35 and FIG. 36 are diagrams illustrating a process for providing a tailor function in a detailed fitting mode according to the present disclosure.

### BEST MODE FOR INVENTION

Description will now be given in detail according to exemplary embodiments disclosed herein, with reference to the accompanying drawings. For the sake of brief description with reference to the drawings, the same or equivalent components may be provided with the same reference numbers, and description thereof will not be repeated. In general, a suffix such as "module" and "unit" may be used to refer to elements or components. Use of such a suffix herein is merely intended to facilitate description of the specification, and the suffix itself is not intended to give any special meaning or function. In the present disclosure, that which is well-known to one of ordinary skill in the relevant art has generally been omitted for the sake of brevity. The accompanying drawings are used to help easily understand various technical features and it should be understood that the embodiments presented herein are not limited by the accompanying drawings. As such, the present disclosure should be construed to extend to any alterations, equivalents and substitutes in addition to those which are particularly set out in the accompanying drawings.

It will be understood that although the terms first, second, etc. may be used herein to describe various elements, these elements should not be limited by these terms. These terms are generally only used to distinguish one element from another.

It will be understood that when an element is referred to as being "connected with or to" another element, the element can be connected with the other element or intervening elements may also be present. In contrast, when an element is referred to as being "directly connected with" another element, there are no intervening elements present.

A singular representation may include a plural representation unless it represents a definitely different meaning from the context.

Terms such as "include" or "has" are used herein and should be understood that they are intended to indicate an existence of several components, functions or steps, disclosed in the specification, and it is also understood that greater or fewer components, functions, or steps may likewise be utilized.

A virtual fitting providing device described in the present specification may have at least one of types such as a smartphone, a laptop computer, a slate PC, a tablet PC, an ultra-book, a digital TV, a desktop computer, a digital signage, and a smart mirror.

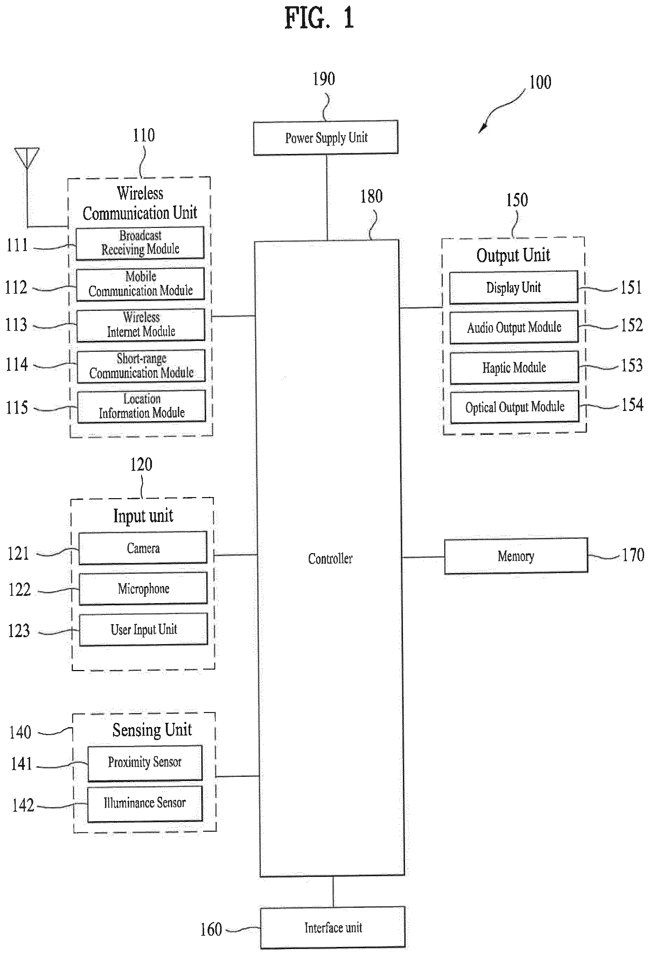

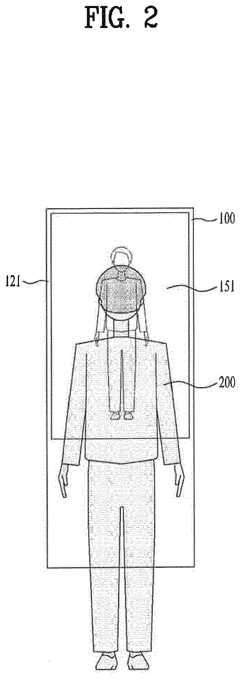

FIG. 1 is a diagram to describe a virtual fitting providing device related to the present disclosure, and FIG. 2 is a conceptual diagram illustrating one example of a virtual fitting providing device related to the present disclosure.

As described in FIG. 1, a virtual fitting providing apparatus (100) may include a wireless communication unit (110), an input unit (120), a sensing unit (140), an output unit (150), an interface unit (160), a memory (170), a controller (180) and a power supply unit (190) and so on. The virtual fitting providing apparatus (100) does not have to all elements described in FIG. 1. Thus, the virtual fitting providing apparatus can have modules smaller or more than elements described in FIG. 1.

Specifically, the wireless communication unit 110 among the above components may include one or more modules between the virtual fitting providing device 100 and a wireless communication system, between the virtual fitting providing device 100 and an external device, and/or between the virtual fitting providing device 100 and a network on which an external service is located.

To facilitate such communications, the wireless communication unit 110 includes one or more of a broadcast receiving module 111, a mobile communication module 112, a wireless Internet module 113, a short-range communication module 114, and a position location module 115.

The input unit 120 includes a camera 121 for obtaining images or video, a microphone 122, which is one type of audio input device for inputting an audio signal, and a user input unit 123 (for example, a touch key, a push key, a mechanical key, a soft key, and the like) for allowing a user to input information. Data (for example, audio, video, image, and the like) is obtained by the input unit 120 and may be analyzed and processed by controller 180 according to device parameters, user commands, and combinations thereof.

The sensing unit 140 is typically implemented using one or more sensors configured to sense internal information of the mobile terminal, the surrounding environment of the mobile terminal, user information, and the like. For example, in FIG. 1A, the sensing unit 140 is shown having a proximity sensor 141 and an illumination sensor 142. If desired, the sensing unit 140 may alternatively or additionally include other types of sensors or devices, such as a touch sensor, an acceleration sensor, a magnetic sensor, a G-sensor, a gyroscope sensor, a motion sensor, an RGB sensor, an infrared (IR) sensor, a finger scan sensor, a ultrasonic sensor, an optical sensor (for example, camera 121), a microphone 122, a battery gauge, an environment sensor (for example, a barometer, a hygrometer, a thermometer, a radiation detection sensor, a thermal sensor, and a gas sensor, among others), and a chemical sensor (for example, an electronic nose, a health care sensor, a biometric sensor, and the like), to name a few. The mobile terminal 100 may be configured to utilize information obtained from sensing unit 140, and in particular, information obtained from one or more sensors of the sensing unit 140, and combinations thereof.

The output unit 150 is typically configured to output various types of information, such as audio, video, tactile output, and the like. The output unit 150 is shown having a display unit 151, an audio output module 152, a haptic module 153, and an optical output module 154. The display unit 151 may have an inter-layered structure or an integrated structure with a touch sensor in order to facilitate a touch screen. The touch screen may provide an output interface between the mobile terminal 100 and a user, as well as function as the user input unit 123 which provides an input interface between the mobile terminal 100 and the user.

The interface unit 160 serves as an interface with various types of external devices that can be coupled to the mobile terminal 100. The interface unit 160, for example, may include any of wired or wireless ports, external power supply ports, wired or wireless data ports, memory card ports, ports for connecting a device having an identification module, audio input/output (I/O) ports, video I/O ports, earphone ports, and the like. In some cases, the mobile terminal 100 may perform assorted control functions associated with a connected external device, in response to the external device being connected to the interface unit 160.

The memory 170 may store a multitude of application programs or applications driven in the virtual fitting providing device 100 and data & instructions for operations of the virtual fitting providing device 100. At least some of the application programs may be downloaded from an external server for wireless communication. At least some of the application programs may exist on the virtual fitting providing device 100 from the time of shipment for a basic virtual fitting service function of the virtual fitting providing device 100. Meanwhile, the application program may be stored in the memory 170, installed on the virtual fitting providing device 100, and driven by the controller 180 to perform an operation (or function) of the virtual fitting providing device 100.

The controller 180 typically functions to control overall operation of the mobile terminal 100, in addition to the operations associated with the application programs. The controller 180 processes signals, data, information and the like inputted or outputted through the above-mentioned components and/or runs application programs saved in the memory 170, thereby processing or providing a user with appropriate information and/or functions.

In addition, the controller 180 may control at least some of the components discussed with reference to FIG. 1 in order to drive an application program stored in the memory 170. Furthermore, in order to drive the application program, the controller 180 may operate at least two or more of the components included in the virtual fitting providing apparatus 100 in combination with each other.

The power supply unit 190 can be configured to receive external power or provide internal power in order to supply appropriate power required for operating elements and components included in the mobile terminal 100. The power supply unit 190 may include a battery, and the battery may be configured to be embedded in the terminal body, or configured to be detachable from the terminal body.

At least one portion of the respective components mentioned in the foregoing description can cooperatively operate to embody operations, controls or controlling methods of the mobile terminal according to various embodiments of the present invention mentioned in the following description. Moreover, the operations, controls or controlling methods of the mobile terminal can be embodied in the mobile terminal by running at least one or more application programs saved in the memory 170.

Hereinafter, before looking at various embodiments implemented through the virtual fitting providing apparatus 100 as described above, the above-listed components will be described in more detail with reference to FIG. 1.

Regarding the wireless communication unit 110, the broadcast receiving module 111 is typically configured to receive a broadcast signal and/or broadcast associated information from an external broadcast managing entity via a broadcast channel. The broadcast channel may include a satellite channel, a terrestrial channel, or both. In some embodiments, two or more broadcast receiving modules 111 may be utilized to facilitate simultaneously receiving of two or more broadcast channels, or to support switching among broadcast channels.

The mobile communication module 112 can transmit and/or receive wireless signals to and from one or more network entities. Typical examples of a network entity include a base station, an external mobile terminal, a server, and the like. Such network entities form part of a mobile communication network, which is constructed according to technical standards or communication methods for mobile communications (for example, Global System for Mobile Communication (GSM), Code Division Multi Access (CDMA), CDMA2000(Code Division Multi Access 2000), EV-DO(Enhanced Voice-Data Optimized or Enhanced Voice-Data Only), Wideband CDMA (WCDMA), High Speed Downlink Packet access (HSDPA), HSUPA(High Speed Uplink Packet Access), Long Term Evolution (LTE) , LTE-A(Long Term Evolution-Advanced), and the like).

The wireless Internet module 113 refers to a module for wireless Internet access, and may be built-in or external to the apparatus 100 for providing virtual fittings. The virtual fitting providing apparatus 100 is configured to transmit and receive a wireless signal in a communication network according to wireless Internet technologies.

Examples of such wireless Internet access include Wireless LAN (WLAN), Wireless Fidelity (Wi-Fi), Wi-Fi Direct, Digital Living Network Alliance (DLNA), Wireless Broadband (WiBro), Worldwide Interoperability for Microwave Access (WiMAX), High Speed Downlink Packet Access (HSDPA), HSUPA(High Speed Uplink Packet Access), Long Term Evolution (LTE), LTE-A(Long Term Evolution-Advanced), and the like. The wireless Internet module 113 may transmit/receive data according to one or more of such wireless Internet technologies, and other Internet technologies as well.

In some embodiments, when the wireless Internet access is implemented according to, for example, WiBro, HSDPA,HSUPA, GSM, CDMA, WCDMA, LTE, LTE-A and the like, as part of a mobile communication network, the wireless Internet module 113 performs such wireless Internet access. As such, the Internet module 113 may cooperate with, or function as, the mobile communication module 112.

The short range communication module 114 is for short range communication, and may support the short range communication using at least one of Bluetooth, Radio Frequency Identification (RFID), Infrared Data Association (IRDA), Ultra Wideband (UWB), Zigbee, Near Field Communication (NFC), and Wireless-Fidelity (Wi-Fi), and Wi-Fi Direct technology. The short-range communication module 114 may support short range wireless communication between the virtual fitting providing device 100 and the wireless communication system, between the virtual fitting providing device 100 and an external device, or between the virtual fitting providing device 100 and an external server.

The location information module 115 is a module for acquiring a location (or current location) of the virtual fitting providing device 100, and a representative example thereof includes a Global Positioning System (GPS) module or a Wireless Fidelity (WIFI) module. For example, when the GPS module is used, the virtual fitting providing apparatus 100 may acquire a location of the virtual fitting providing apparatus 100 by using a signal transmitted from a GPS satellite. For another example, when using a Wi-Fi module, the virtual fitting providing apparatus 100 may acquire a location of a mobile terminal based on information on a wireless Access Point (AP) that transmits or receives wireless signals to or from the Wi-Fi module.

The input unit 120 may be configured to permit various types of input to the mobile terminal 100. Examples of such input include audio, image, video, data, and user input. Image and video input is often obtained using one or more cameras 121. Such cameras 121 may process image frames of still pictures or video obtained by image sensors in a video or image capture mode. The processed image frames can be displayed on the display unit 151 or stored in memory 170. In some cases, the cameras 121 may be arranged in a matrix configuration to permit a plurality of images having various angles or focal points to be input to the mobile terminal 100. As another example, the cameras 121 may be located in a stereoscopic arrangement to acquire left and right images for implementing a stereoscopic image.

The microphone 122 is generally implemented to permit audio input to the mobile terminal 100. The audio input can be processed in various manners according to a function being executed in the mobile terminal 100. If desired, the microphone 122 may include assorted noise removing algorithms to remove unwanted noise generated in the course of receiving the external audio.

The user input unit 123 is a component that permits input by a user. Such user input may enable the controller 180 to control operation of the mobile terminal 100. The user input unit 123 may include one or more of a mechanical input element (for example, a key, a button located on a front and/or rear surface or a side surface of the mobile terminal 100, a dome switch, a jog wheel, a jog switch, and the like), or a touch-sensitive input, among others. As one example, the touch-sensitive input may be a virtual key or a soft key, which is displayed on a touch screen through software processing, or a touch key which is located on the mobile terminal at a location that is other than the touch screen. On the other hand, the virtual key or the visual key may be displayed on the touch screen in various shapes, for example, graphic, text, icon, video, or a combination thereof.

The sensing unit 140 is generally configured to sense one or more of internal information of the mobile terminal, surrounding environment information of the mobile terminal, user information, or the like. The controller 180 generally cooperates with the sending unit 140 to control operation of the mobile terminal 100 or execute data processing, a function or an operation associated with an application program installed in the mobile terminal based on the sensing provided by the sensing unit 140. The sensing unit 140 may be implemented using any of a variety of sensors, some of which will now be described in more detail.

The proximity sensor 141 may include a sensor to sense presence or absence of an object approaching a surface, or an object located near a surface, by using an electromagnetic field, infrared rays, or the like without a mechanical contact. The proximity sensor 141 may be arranged at an inner region of the mobile terminal covered by the touch screen, or near the touch screen.

The proximity sensor 141, for example, may include any of a transmissive type photoelectric sensor, a direct reflective type photoelectric sensor, a mirror reflective type photoelectric sensor, a high-frequency oscillation proximity sensor, a capacitance type proximity sensor, a magnetic type proximity sensor, an infrared rays proximity sensor, and the like. When the touch screen is implemented as a capacitance type, the proximity sensor 141 can sense proximity of a pointer relative to the touch screen by changes of an electromagnetic field, which is responsive to an approach of an object with conductivity. In this case, the touch screen (touch sensor) may also be categorized as a proximity sensor.

The proximity sensor 141, for example, may include any of a transmissive type photoelectric sensor, a direct reflective type photoelectric sensor, a mirror reflective type photoelectric sensor, a high-frequency oscillation proximity sensor, a capacitance type proximity sensor, a magnetic type proximity sensor, an infrared rays proximity sensor, and the like. When the touch screen is implemented as a capacitance type, the proximity sensor 141 can sense proximity of a pointer relative to the touch screen by changes of an electromagnetic field, which is responsive to an approach of an object with conductivity. In this case, the touch screen (touch sensor) may also be categorized as a proximity sensor. The term "proximity touch" will often be referred to herein to denote the scenario in which a pointer is positioned to be proximate to the touch screen without contacting the touch screen. The term "contact touch" will often be referred to herein to denote the scenario in which a pointer makes physical contact with the touch screen. For the position corresponding to the proximity touch of the pointer relative to the touch screen, such position will correspond to a position where the pointer is perpendicular to the touch screen. The proximity sensor 141 may sense proximity touch, and proximity touch patterns (for example, distance, direction, speed, time, position, moving status, and the like). In general, controller 180 processes data corresponding to proximity touches and proximity touch patterns sensed by the proximity sensor 141, and cause output of visual information on the touch screen. In addition, the controller 180 can control the mobile terminal 100 to execute different operations or process different data according to whether a touch with respect to a point on the touch screen is either a proximity touch or a contact touch.

A touch sensor can sense a touch applied to the touch screen, such as display unit 151, using any of a variety of touch methods. Examples of such touch methods include a resistive type, a capacitive type, an infrared type, and a magnetic field type, among others.

As one example, the touch sensor may be configured to convert changes of pressure applied to a specific part of the display unit 151, or convert capacitance occurring at a specific part of the display unit 151, into electric input signals. The touch sensor may also be configured to sense not only a touched position and a touched area, but also touch pressure and/or touch capacitance. A touch object is generally used to apply a touch input to the touch sensor. Examples of typical touch objects include a finger, a touch pen, a stylus pen, a pointer, or the like.

When a touch input is sensed by a touch sensor, corresponding signals may be transmitted to a touch controller. The touch controller may process the received signals, and then transmit corresponding data to the controller 180. Accordingly, the controller 180 may sense which region of the display unit 151 has been touched. Here, the touch controller may be a component separate from the controller 180, the controller 180, and combinations thereof.

In some embodiments, the controller 180 may execute the same or different controls according to a type of touch object that touches the touch screen or a touch key provided in addition to the touch screen. Whether to execute the same or different control according to the object which provides a touch input may be decided based on a current operating state of the mobile terminal 100 or a currently executed application program, for example.

The touch sensor and the proximity sensor may be implemented individually, or in combination, to sense various types of touches. Such touches includes a short (or tap) touch, a long touch, a multi-touch, a drag touch, a flick touch, a pinch-in touch, a pinch-out touch, a swipe touch, a hovering touch, and the like.

If desired, an ultrasonic sensor may be implemented to recognize position information relating to a touch object using ultrasonic waves. The controller 180, for example, may calculate a position of a wave generation source based on information sensed by an illumination sensor and a plurality of ultrasonic sensors. Since light is much faster than ultrasonic waves, the time for which the light reaches the optical sensor is much shorter than the time for which the ultrasonic wave reaches the ultrasonic sensor. The position of the wave generation source may be calculated using this fact. For instance, the position of the wave generation source may be calculated using the time difference from the time that the ultrasonic wave reaches the sensor based on the light as a reference signal.

The camera 121 typically includes at least one a camera sensor (CCD, CMOS etc.), a photo sensor (or image sensors), and a laser sensor.

Implementing the camera 121 with a laser sensor may allow detection of a touch of a physical object with respect to a 3D stereoscopic image. The photo sensor may be laminated on, or overlapped with, the display device. The photo sensor may be configured to scan movement of the physical object in proximity to the touch screen. In more detail, the photo sensor may include photo diodes and transistors at rows and columns to scan content received at the photo sensor using an electrical signal which changes according to the quantity of applied light. Namely, the photo sensor may calculate the coordinates of the physical object according to variation of light to thus obtain position information of the physical object.

The display unit 151 is generally configured to output information processed in the mobile terminal 100. For example, the display unit 151 may display execution screen information of an application program executing at the mobile terminal 100 or user interface (UI) and graphic user interface (GUI) information in response to the execution screen information.

In some embodiments, the display unit 151 may be implemented as a stereoscopic display unit for displaying stereoscopic images.

A typical stereoscopic display unit may employ a stereoscopic display scheme such as a stereoscopic scheme (a glass scheme), an auto-stereoscopic scheme (glassless scheme), a projection scheme (holographic scheme), or the like.

An audio output unit 152 may output audio data received from the wireless communication unit 110 or stored in the memory 170. The audio output unit 152 may output a sound signal related to a virtual fitting service performed by the virtual fitting providing device 100. The audio output unit 152 may include a receiver, a speaker, a buzzer, and the like.

A haptic module 153 can be configured to generate various tactile effects that a user feels, perceive, or otherwise experience. A typical example of a tactile effect generated by the haptic module 153 is vibration. The strength, pattern and the like of the vibration generated by the haptic module 153 can be controlled by user selection or setting by the controller. For example, the haptic module 153 may output different vibrations in a combining manner or a sequential manner.

Besides vibration, the haptic module 153 can generate various other tactile effects, including an effect by stimulation such as a pin arrangement vertically moving to contact skin, a spray force or suction force of air through a jet orifice or a suction opening, a touch to the skin, a contact of an electrode, electrostatic force, an effect by reproducing the sense of cold and warmth using an element that can absorb or generate heat, and the like.

The haptic module 153 can also be implemented to allow the user to feel a tactile effect through a muscle sensation such as the user's fingers or arm, as well as transferring the tactile effect through direct contact. Two or more haptic modules 153 may be provided according to the particular configuration of the mobile terminal 100.

An optical output unit 154 outputs a signal for notifying occurrence of an event using light from a light source of the virtual fitting providing apparatus 100.

An optical output module 154 can output a signal for indicating an event generation using light of a light source. Examples of events generated in the mobile terminal 100 may include message reception, call signal reception, a missed call, an alarm, a schedule notice, an email reception, information reception through an application, and the like.

The interface unit 160 serves as an interface for external devices to be connected with the mobile terminal 100. For example, the interface unit 160 can receive data transmitted from an external device, receive power to transfer to elements and components within the mobile terminal 100, or transmit internal data of the mobile terminal 100 to such external device. The interface unit 160 may include wired or wireless headset ports, external power supply ports, wired or wireless data ports, memory card ports, ports for connecting a device having an identification module, audio input/output (I/O) ports, video I/O ports, earphone ports, or the like.

The memory 170 can store programs to support operations of the controller 180 and store input/output data (for example, phonebook, messages, still images, videos, etc.). The memory 170 may store data related to various patterns of vibrations and audio which are output in response to touch inputs on the touch screen.

The memory 170 may include at least one of types of storage media such as a flash memory type, a hard disk type, a multimedia card micro type, a card type memory (e.g., SD or XD memory, etc.), a Random Access Memory (RAM), a Static Random Access Memory (SRAM), a Read-Only Memory (ROM), an Electrically Erasable Programmable Read-Only Memory (EEPROM), Programmable Read-Only Memory (PROM), a magnetic memory, a magnetic disk, and an optical disk..

Meanwhile, as described above, the controller 180 controls operations related to application programs and overall operations of the virtual fitting providing device 100 in general.

In addition, the controller 180 may control one or a plurality of the above-described components in combination to implement various embodiments described below on the virtual fitting providing device 100 according to the present disclosure.

The power supply unit 190 receives external power and/or internal power under the control of the controller 180 to supply power necessary for the operation of each component. The power supply unit 190 includes a battery, and the battery may be a rechargeable built-in battery, and may be detachably coupled to a terminal body for charging and the like.

Meanwhile, various embodiments below may be implemented in a computer or similar device-readable recording medium using, for example, software, hardware, or a combination thereof.

FIG. 2 illustrates one example of a virtual fitting providing device related to the present disclosure.

Referring to FIG. 2, a subject 200 corresponding to a user is located on the virtual fitting providing device 100, and the display unit 151 may be configured as a mirror that shows an appearance of the subject 200 while a screen is turned off.

If detecting that the subject 200 is located in a preset distance in front via the camera 121, the controller 180 may activate a virtual fitting mode for providing a virtual fitting service according to the present disclosure and may perform a virtual fitting providing operation described below.

In some implementations, the camera 121 may include a #D camera capable of measurement of a distance between the virtual fitting providing device 100 and the subject 200, scanning of a face of the subject, and measurement of a body size of the subject 200. The 3D camera may measure the body size of the subject 200 by at least one of a stereo system, a structured light system, and a Time Of Flight (TOF) system.

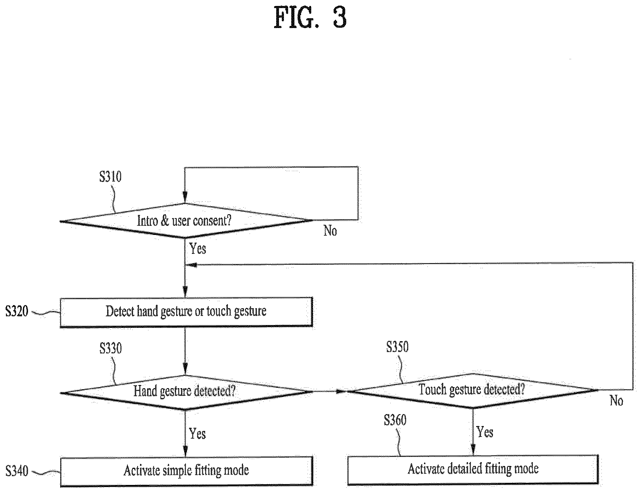

Hereinafter, a process for providing a virtual fitting according to the present disclosure will be described in detail with reference to FIGs. 3 to 36.

First of all, FIG. 3 is a flowchart illustrating a virtual fitting providing process according to the present disclosure.

Referring to FIG. 3, when the subject 200 is detected as located within a predetermined distance in front via the camera 121, the controller 180 activates a virtual fitting mode for providing a virtual fitting service according to the present disclosure, and displays an intro screen 610 for introduction and user consent for the virtual fitting service on the touchscreen 151.

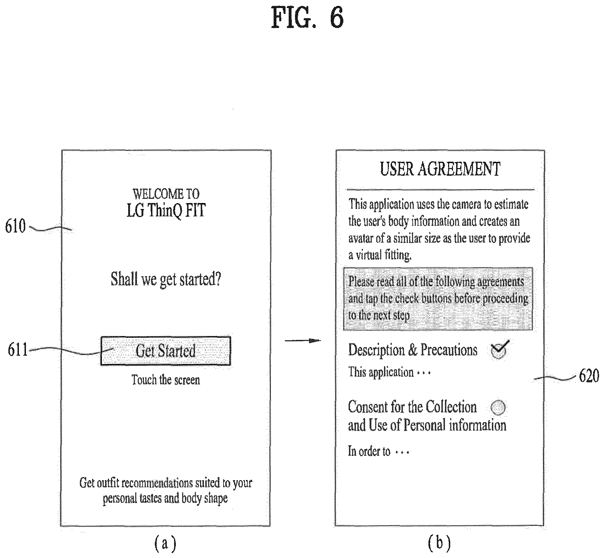

FIG. 6 is a diagram to describe an intro screen of a virtual fitting service according to the present disclosure.

As shown in FIG. 6 (a), the intro screen 610 includes a start menu 611 for starting a virtual fitting service according to the present disclosure. If the start menu 611 is selected, as shown in FIG. 6 (b), the controller 180 displays an consent setting window 620 for setting whether to give user consent to the virtual fitting service.

If the user consent is set through the consent setting window 620 [S310], it is detected whether a preset hand gesture of the subject 200 is inputted via the camera 121 or whether a preset touch gesture of the subject 200 is inputted onto the touchscreen 151 [S320].

As a result of the detection, if the preset hand gesture of the subject 200 is detected as inputted based on an image of the subject 200 captured via the camera 121 [S330]. The controller 180 activates a simple fitting mode [S340].

Here, the simple fitting mode means a hand gesture based mode in which the subject 200 may manipulate the virtual fitting service conveniently using a hand gesture.

In some implementations, the preset hand gesture may include a hand gesture in a state in which the subject 200 opens a palm toward a screen of the touchscreen 151.

In addition, as a result of the detection, if the preset touch gesture of the subject 200 is detected as inputted onto the touchscreen 151 [S350], the controller 180 activates a detailed fitting mode [S360].

Here, the detailed fitting mode means a touch gesture based mode in which the subject 200 may manipulate the virtual fitting service in detail using a touch gesture on the touchscreen 151.

In some implementations, the preset touch gesture may include a gesture of touching the screen of the touchscreen 151 by the subject 200.

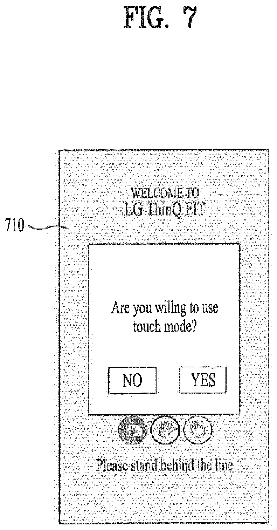

FIG. 7 is a diagram to describe a process for switching from a simple fitting mode to a detailed fitting mode according to the present disclosure.

Referring to FIG. 7, a user may switch a current simple fitting mode based on a hand gesture to a detailed fitting mode via a touch gesture. Namely, if a screen of the touchscreen 151 is touched in the simple fitting mode, the controller 180 displays an inquiry window 710 inquiring whether to switch a current simple fitting mode to a detailed fitting mode based on a touch gesture. If the switch to the detailed fitting mode is set through the inquiry window 710, the controller may directly switch the simple fitting mode to the detailed fitting mode.

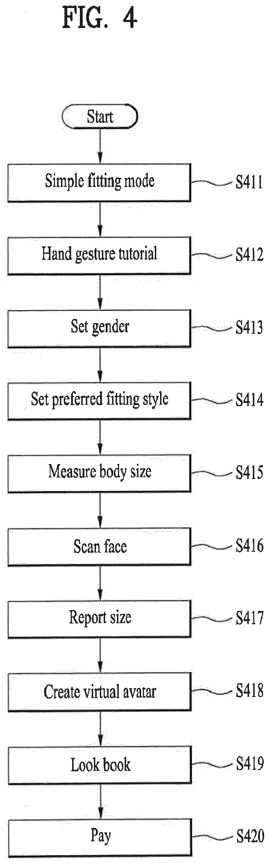

FIG. 4 is a flowchart illustrating an operating process of a simple fitting mode of a virtual fitting service according to the present disclosure.

Referring to FIG. 4, if the simple fitting mode is activated by the process of FIG. 3 [S411], the controller 180 displays a hand gesture tutorial screen, which informs the subject 200 of two or more hand gestures for the manipulation of the virtual fitting service, on the touchscreen 151 [S412].

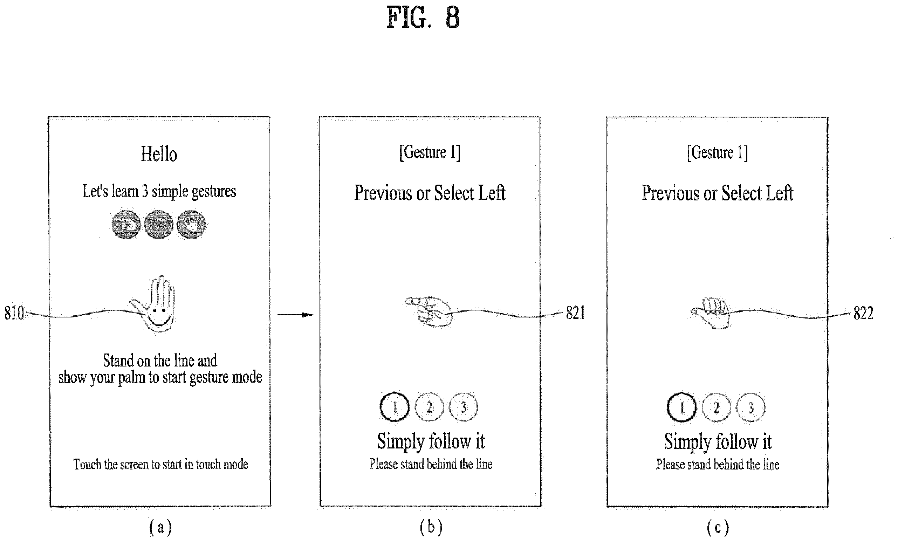

Hereinafter, hand gestures available for the subject 200 through the hand gesture tutorial screen in the simple fitting mode will be described with reference to FIGs. 8 to 10.

FIGs. 8 to 10 are diagrams to describe a hand gesture tutorial screen in a simple fitting mode according to the present disclosure.

First of all, FIG. 8 (a) shows that a first gesture guide 810 guiding an input of a first hand gesture to activate a hand gesture based simple fitting mode is displayed on a hand gesture tutorial screen.

For example, the first hand gesture may include a hand gesture in a state that the subject 200 opens a palm in a direction toward the camera 121. The subject 200 follows the first hand gesture according to the first gesture guide 810 to activate the simple fitting mode.

Namely, if recognizing that the subject 200 has taken the first hand gesture based on an image of the subject 200 received through the camera 121, the controller 180 directly activates the simple fitting mode.

FIG. 8 (b) shows a second gesture guide 821 guiding an input of a 2-1 hand gesture for moving to a previous page from a current page within the hand gesture tutorial screen or selecting an object located on a left side within the screen. In this case, the object is an object selectable from the screen. If a specific function is assigned to the object, the function assigned to the object may be executed in response to a selection of the object.

For example, the 2-1 hand gesture may include a hand gesture in which the subject 200 points an index finger of a right hand to a left side, and the subject 200 may follow the 2-1 hand gesture to move a current page in the screen to a previous page or select an item located on the left side in the screen.

Namely, if recognizing that the subject has taken the 2-1 hand gesture based on an image of the subject 200 received through the camera 121, the controller 180 may move the current page in the screen to the previous pate or select the item located on the left side in the screen.

A third gesture guide 822 shown in FIG. 8 (c) is an item for guiding an input of a 2-2 hand gesture for moving a current page in the hand gesture tutorial screen to a previous page or select an object located on a left side in the screen, and the 2-2 hand gesture includes a hand gesture in which the subject 200 points a thumb of a left hand to a left side.

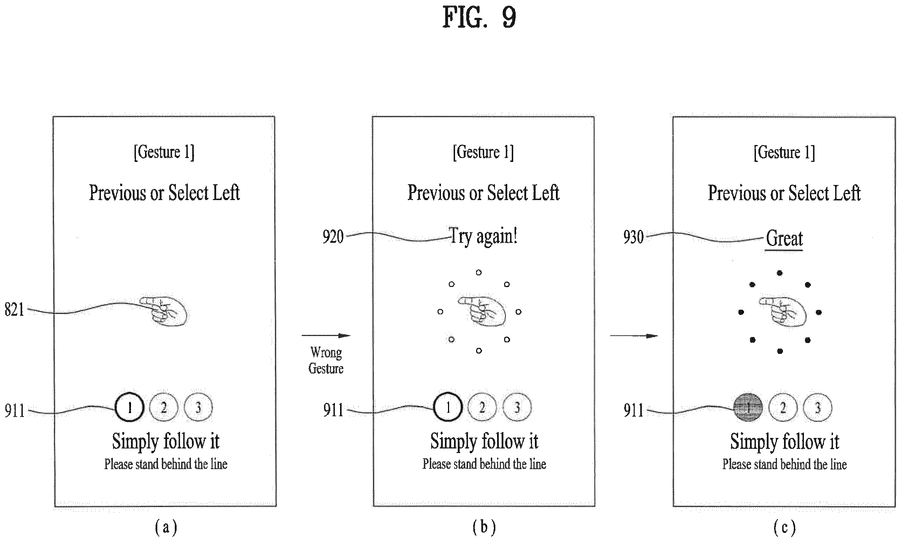

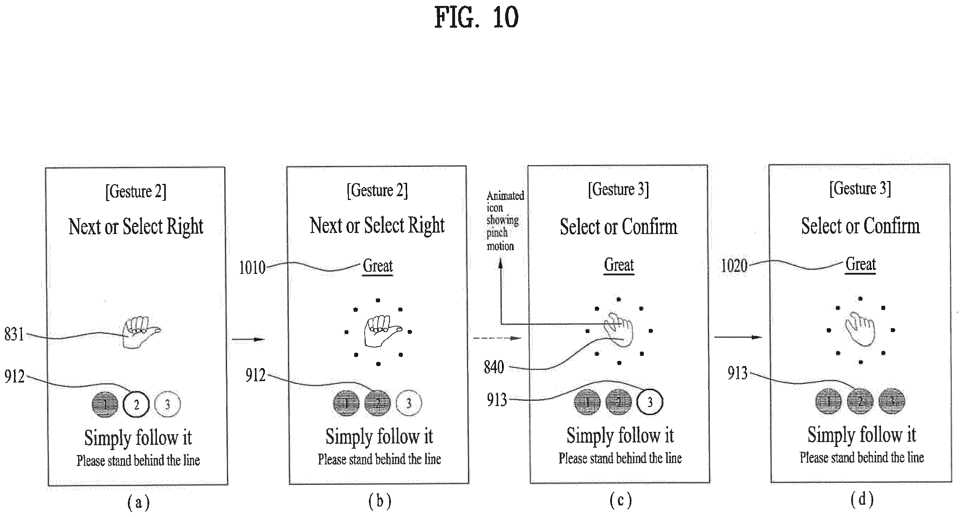

FIG. 9 and FIG. 10 show a process for feeding recognition failure/success in hand gesture recognition back to the subject 200.

First of all, as shown in FIG. 9 (a), while displaying the second gesture guide 821 in the hand gesture tutorial screen, the controller 180 recognizes whether the 2-1 hand gesture related to the second gesture guide 821 is inputted through the camera 121.

In doing so, the controller 180 may display a first indicator 911 indicating that a current page in the hand gesture tutorial screen relates to a process for recognizing the 2-1 hand gesture.

If failing in the recognition of the 2-1 hand gesture inputted from the subject 200, as shown in FIG. 9 (b), the controller 180 displays a first visual feedback 920 indicating that the failure in the recognition of the 2-1 hand gesture.

The first visual feedback 920 may include a text ("Try again!") and animation to indicate the failure in the recognition of the 2-1 hand gesture and guide a re-input of the 2-1 hand gesture. The controller 180 may display the first visual feedback 920 until succeeding in the recognition of the 2-1 hand gesture from the subject 200.

In addition, based on the image of the subject 200 received through the camera 121, if the subject 200 does not take a hand gesture for a preset time, puts a hand down at all, or takes no additional hand gesture, the controller 180 may stop displaying the first visual feedback 920.

Moreover, in addition to the first visual feedback 920, the controller 180 may further output a first auditory feedback for the same purpose as the first visual feedback 920.

Meanwhile, when the recognition of the 2-1 hand gesture input from the subject 200 is successful, as shown in FIG. 9 (c), the controller 180 displays a second visual feedback 930 indicating that the recognition of the 2-1 hand gesture is successful.

The second visual feedback 930 may include text ("Great") and animation indicating that recognition of the 2-1 hand gesture is successful. In addition, the controller 180 may further output a second auditory feedback for the same purpose as the second visual feedback 930, in addition to the second visual feedback 930.

In addition, the controller 180 may display the first indicator 911 in a first display style until the 2-1 hand gesture is successfully recognized, and may display the first indicator 911 in a second display style after the 2-1 hand gesture is successfully recognized.

For example, the first display style may include a style in which an outline of the first indicator 911 is displayed in a first color, and the second display style may include a style in which the entire first indicator 911 is displayed in a manner of being filled in the first color.

Next, FIG. 10 (a) shows a fourth gesture guide 831 for guiding an input of a third hand gesture for moving a current page to a next page in a hand gesture tutorial screen or selecting an object located on the right side in the screen.

The controller 180 recognizes whether the third hand gesture related to the fourth gesture guide 831 is inputted through the camera 121 while displaying the fourth gesture guide 831. For example, the third hand gesture may include a hand gesture in the form of pointing a thumb of a right hand to the right or a hand gesture in the form of pointing an index finger of a left hand to the right.

In addition, the controller 180 may display a second indicator 912 indicating that a current page in the hand gesture tutorial screen is a recognition process of the third hand gesture.

When recognition of the third hand gesture inputted from the subject 200 fails, the controller 180 displays a third visual feedback indicating that recognition of the third hand gesture fails.

The third visual feedback may include a text ("Try again!") and animation to indicate the failure in recognizing the third hand gesture and guide the re-input of the third hand gesture, and the controller 180 may display the third visual feedback until succeeding in recognizing the third hand gesture from the subject 200.

In addition, based on the image of the subject 200 received through the camera 121, if the subject 200 does not take a hand gesture for a preset time, puts a hand down at all, or takes no additional hand gesture, the controller 180 may stop displaying the third visual feedback.

Moreover, in addition to the third visual feedback, the controller 180 may further output a third auditory feedback for the same purpose as the third visual feedback 920.

Meanwhile, when the recognition of the third hand gesture inputted from the subject 200 is successful, as shown in FIG. 10 (c), the controller 180 displays a fourth visual feedback 1010 indicating that the recognition of the third hand gesture is successful.

The fourth visual feedback 1010 may include a text ("Great") and animation indicating that recognition of the third hand gesture is successful. In addition, the controller 180 may further output a fourth auditory feedback for the same purpose as the fourth visual feedback 1010, in addition to the fourth visual feedback 1010.

In addition, the controller 180 may display the second indicator 912 in a first display style until the third hand gesture is successfully recognized, and may display the second indicator 912 in a second display style after the third hand gesture is successfully recognized.

For example, the first display style may include a style in which an outline of the second indicator 912 is displayed in a first color, and the second display style may include a style in which the entire second indicator 912 is displayed in a manner of being filled in the first color.

Next, FIG. 10 (c) shows a fifth hand gesture 840 guiding an input of a fourth hand gesture for object selection or information confirmation in the hand gesture tutorial screen.

In a state in which the fifth gesture guide 840 is displayed, the controller 180 recognizes whether the fourth hand gesture related to the fifth gesture guide 840 is inputted through the camera 121. For example, the fourth hand gesture may include a pinch-in hand gesture in which the index finger and thumb of the subject 200 are folded and then released.

In addition, the controller 180 may display a third indicator 913 indicating that a current page in the hand gesture tutorial screen is a recognition process of the fourth hand gesture.

When the recognition of the fourth hand gesture inputted from the subject 200 fails, the controller 180 displays a fifth visual feedback indicating that the recognition of the fourth hand gesture fails.

The fifth visual feedback may include a text ("Try again!") and animation to indicate the failure in recognizing the fourth hand gesture and guide the re-input of the fourth hand gesture, and the controller 180 may display the fifth visual feedback until succeeding in recognizing the fourth hand gesture from the subject 200.

In addition, based on the image of the subject 200 received through the camera 121, if the subject 200 does not take a hand gesture for a preset time, puts a hand down at all, or takes no additional hand gesture, the controller 180 may stop displaying the fifth visual feedback.

Moreover, in addition to the fifth visual feedback, the controller 180 may further output a fifth auditory feedback for the same purpose as the fifth visual feedback 920.

Meanwhile, when the recognition of the fourth hand gesture inputted from the subject 200 is successful, as shown in FIG. 10 (d), the controller 180 displays a sixth visual feedback 1020 indicating that the recognition of the fourth hand gesture is successful.

The fourth visual feedback 1020 may include a text ("Great") and animation indicating that recognition of the fourth hand gesture is successful. In addition, the controller 180 may further output a sixth auditory feedback for the same purpose as the sixth visual feedback 1020, in addition to the sixth visual feedback 1020.

In addition, the controller 180 may display the third indicator 913 in a first display style until the fourth hand gesture is successfully recognized, and may display the third indicator 913 in a second display style after the fourth hand gesture is successfully recognized.

For example, the first display style may include a style in which an outline of the third indicator 913 is displayed in a first color, and the second display style may include a style in which the entire third indicator 913 is displayed in a manner of being filled in the first color.

Referring back to FIG. 4, the controller 180 displays a setting screen for receiving a gender setting from the subject 200 and receives a gender setting of the subject 200 from the subject 200 through the setting screen [S413].

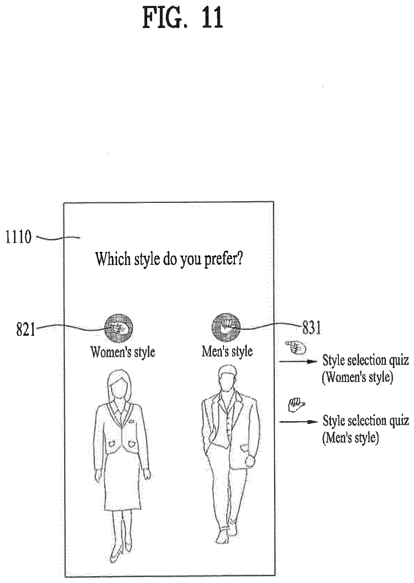

FIG. 11 is a diagram to describe a process for setting a gender of a subject in a simple fitting mode according to the present disclosure.

The step S413 is described with reference to FIG. 11 as follows. First of all, since all screen manipulations are performed by hand gestures of the subject 200 in the simple fitting mode, the controller 180 displays a second gesture guide 821 for guiding an input of a 2-1 hand gesture for selecting a female gender and a fourth gesture guide 831 for selecting a male gender in the setting screen 1110.

If detecting the input of the 2-1 hand gesture, the controller 180 recognizes that the gender of the subject 200 is set to female. If detecting the input of the third hand gesture, the controller 180 recognizes that the gender of the subject 200 is set to male.

Referring back to FIG. 4, once the gender of the subject 200 is set, the controller 180 displays a setting screen for receiving a setting of at least one preferred fitting style according to the gender set by the subject 200 and receives a setting of at least one preferred fitting style from the subject 200 through the setting screen [S414].

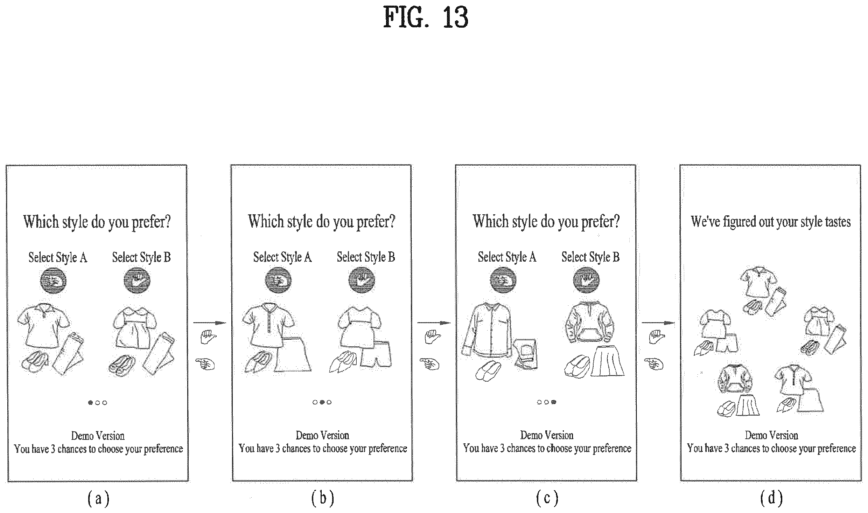

FIG. 13 is a diagram to describe a process for setting a preferred fitting style in a simple fitting mode according to the present disclosure.

FIGs. 13 (a) to 13 (c) show pages of a screen including a multitude of preferred fitting styles for setting at least one preferred fitting style of the subject 200, respectively, and the subject 200 may set at least one preferred fitting style desired in each page using the 2-1 hand gesture or the third hand gesture.

In addition, as illustrated in FIG. 13 (d), the controller 180 may search and display at least one fashion item related to the at least one preferred fitting style set in FIGs. 13 (a) to 13 (c).

In this case, the controller 180 may search for at least one fashion item related to the at least one preferred fitting style from information on fashion items previously stored in the memory 170, or may access a preset external fashion item providing server to search for at least one fashion item related to the at least one preferred fitting style.

Moreover, the at least one fashion item may include at least one of at least one outfit (top and bottom), shoes, and accessory.

Referring back to FIG. 4, the controller 180 measures a body size of the subject 200 [S415].

In this case, the camera 121 of the present disclosure may include a 3D camera capable of measuring a distance between the virtual fitting providing device 100 and the subject 200, scanning a face of the subject 200 and measuring a body size of the subject 200. The 3D camera may measure the body size of the subject 200 by at least one of a stereo method, a structural light method, and a Time Of Flight (TOF) method.

Namely, the controller 180 may measure a detailed body size of the subject 200 through the 3D camera. In this case, the body size measurement process of the subject 200 is the same for both the simple fitting mode and the detailed fitting mode.

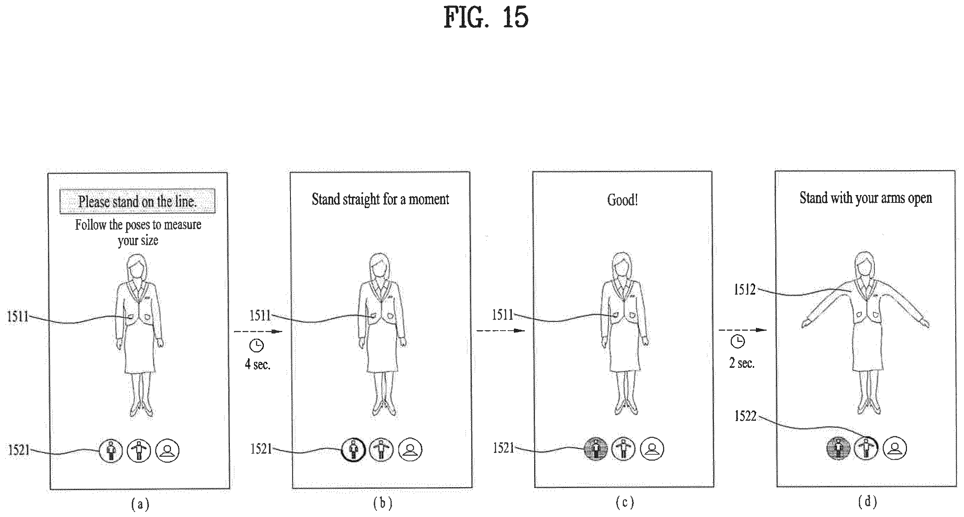

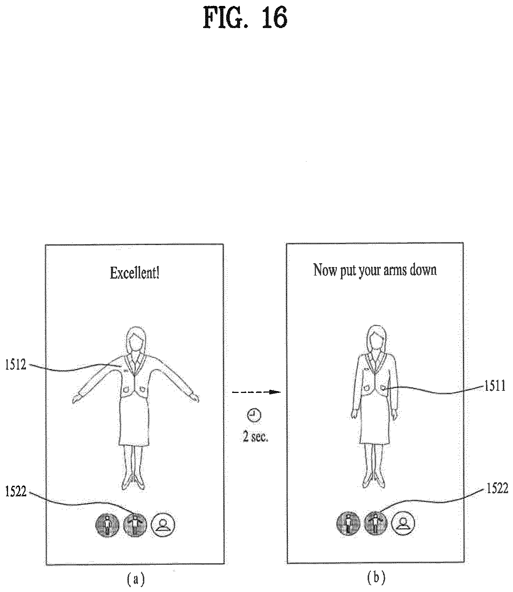

FIG. 15 and FIG. 16 are diagrams to describe a process for measuring a subject body size in a simple/detailed fitting mode according to the present disclosure.

Referring to FIG. 15 and FIG. 16, the controller 180 may make a request for two or more body postures to the subject 200, thereby measuring the body size of the subject 200 in detail. In this case, the controller 180 may display guide information for enabling the subject 200 to follow the body postures.

First of all, as shown in FIG. 15 (a), before measuring the body size of the subject 200, the controller 180 displays a first posture guide 1511 guiding an input of a first body posture among the two or more body posture in a body size measurement screen.

In addition, the controller 180 may display a first posture indicator 1521, which indicates that a current page in the body size measurement screen is a recognition process of the first body posture among the two or more body postures, in the body size measurement screen.

Next, as shown in FIG. 15 (b), the controller 180 recognizes whether the first body posture related to the first posture guide 1511 is inputted from the subject 200 within a preset time through the 3D camera in a state of displaying the first posture guide 1511. For example, the first body posture may include an erect postu re.

Meanwhile, as shown in FIG. 15 (b), the controller 180 may apply a visual effect indicating that the first body posture of the subject 200 is being recognized to the first posture indicator 1521.

Next, as shown in FIG. 15 (c), when the recognition of the first body posture inputted from the subject 200 is successful within a preset time, the controller 180 may apply a visual effect indicating the recognition success of the first body posture to the first posture indicator 1521.

In addition, if the first body posture is recognized, the controller 180 measures and stores a first body size of two or more body parts of the subject 200 according to the first body posture through the 3D camera. In this case, the first body size may include at least one of a total height, weight, shoulder width, neck circumference, shoulder, chest, waist, hip, arm length, and leg length.

Next, as shown in FIG. 15 (d), the controller 180 displays a second posture guide 1512, which guides an input of a second body posture among the two or more body postures, in the body size measurement screen.

In addition, the controller 180 displays a second posture indicator 1522 indicating that a current page in the body size measurement screen is a recognition process of the second body posture among the two or more body postures, and recognizes whether a second posture related to the second posture guide 1512 is inputted from the subject 200 within a preset time. For example, the second body posture may include a posture in which both arms are open.

Meanwhile, as shown in FIG. 15 (d), the controller 180 may apply a visual effect, which indicates that the second body posture of the subject 200 is being recognized, to the second posture indicator 1522.

Next, as shown in FIG. 16 (a), if the recognition of the second body posture inputted from the subject 200 is successful within a preset time, the controller 180 may apply a visual effect, which indicates the recognition success of the second body posture, to the second posture indicator 1522.

In addition, if the second body posture is recognized, the controller 180 measures and stores a second body size of two or more body parts of the subject 200 according to the second body posture through the 3D camera. In this case, the second body size may include at least one of a total height, weight, shoulder width, neck circumference, shoulder, chest, waist, hip, arm length, and leg length.

As described above, if both of the first and second body sizes are completely measured, the controller 180 may determine an average value of the first and second body sizes as a final body size of the subject 200.

In addition, as shown in FIG. 16 (b), if the measurement of the body size of the subject 200 is completed, the controller 180 displays the first posture guide 1511, which guides the second body posture taken by the subject 200 to return to the original first body posture, in the body size measurement screen.

Referring back to FIG. 4, the controller 180 scans a face of the subject 200 through the 3D camera [S416]. In this case, the face scanning process of the subject 200 is the same for both the simple fitting mode and the detailed fitting mode.

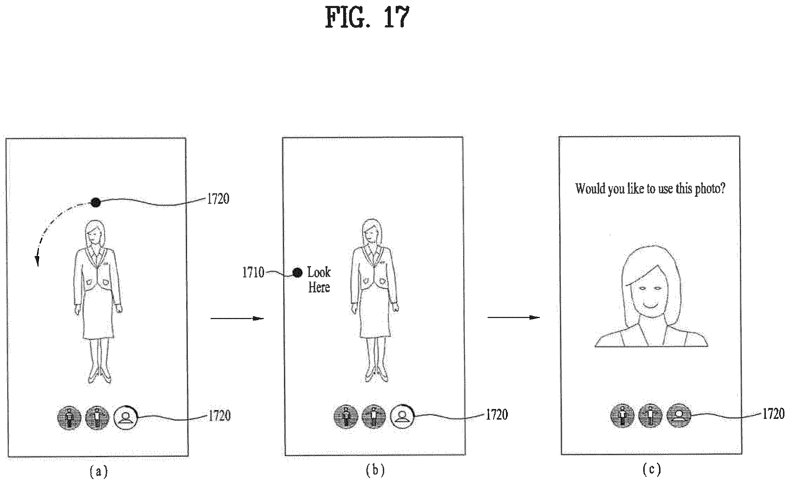

FIG. 17 is a diagram to describe a process for scanning a subject face in a simple/detailed fitting mode according to the present disclosure.

Referring to FIG. 17, the controller 180 may scan a face of a subject by requesting the subject to move a head in a specific pattern. That is, the controller 180 may display guide information, which guides the subject 200 to move the head in the specific pattern, in a face scanning screen.

As shown in FIG. 17 (a), the controller 180 displays a head motion guide 1710, which guides the subject 200 to move the head in a specific pattern, in the face scanning screen.

In this case, the head motion guide 1710 may guide the head of the subject 200 to move in a left or right direction from a front side.

Namely, the head motion guide 1710 is displayed to be automatically moved in the left or right direction from the front side in the face scanning screen, the subject 200 moves its head in the left or right direction from the front side according to the movement of the head motion guide 1710, and the controller 180 scans the face of the currently moving subject 200 through the camera 121.

Meanwhile, the controller 180 may display a scanning indicator 1720, which indicates that a face scanning process is in progress in the face scanning screen, in the body size measurement screen.

Next, as shown in FIG. 17 (b), if the face scanning of the subject 200 is completed within a preset time, the controller 180 displays the face of the subject 200 that has been finally scanned in the form of a virtual avatar as shown in FIG. 17 (c).

In this case, as shown in FIG. 17 (a) and FIG. 17 (b), the controller 180 may apply a visual effect, which indicates that the face of the subject 200 is being scanned, to the scanning indicator 1720.

In addition, as shown in FIG. 17 (c), the controller 180 may apply a visual effect, which indicates that face scanning of the subject 200 is completed, to the scanning indicator 1720.

Referring back to FIG. 4, if the body size measurement and face scanning of the subject 200 are completed, the controller 180 displays a report result screen for the measured body size of the subject 200 [S417].

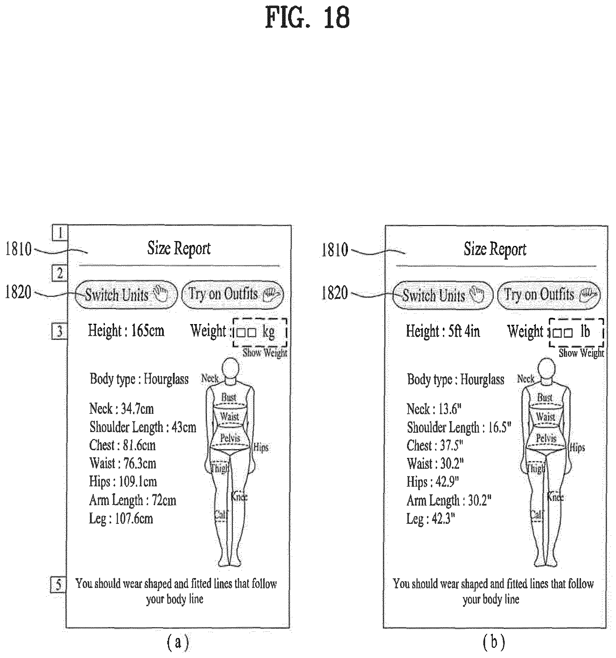

FIG. 18 is a diagram illustrating a subject's body size report screen provided in a simple fitting mode according to the present disclosure.

Referring to FIG. 18, a body size report screen 1810 may represent the total height, weight, shoulder width, neck circumference, shoulder, chest, waist, hip, arm length, and leg length of the subject 200.

In this case, the subject 200 may select the unit change menu 1820 through the fourth hand gesture described above to change the unit for the body sizes.

For example, in FIG. 18(a), the body sizes of the subject 200 are shown in units of centimeters (cm) and kilograms (kg). In FIG. 18 (b), the body sizes of the subject 200 are shown in units of inches and pounds (lbs). The controller 180 may switch the centimeter (cm) and kilogram (kg) units to inch and pound (lb) units whenever a unit switch menu 1820 is selected from the subject 200.

Meanwhile, in FIG. 18, a square box (1) area indicates an area in which a title of the body size report screen is displayed. A square box (2) area indicates an area in which gesture guides for guiding inputs of hand gestures to select or execute menus in the body size report screen. A square box (3) area indicates an area in which body sizes of the subject 200 are displayed. A square box (4) area indicates an area in which weight of the subject 200 is displayed, but is not displayed until it is touched by the subject 200. A square box (5) area indicates an area in which description of body type definition based on the measured body sizes and gender is displayed.

In this case, the body type includes at least one of an inverted triangle body type, a straight body type, a pear body type, and an hourglass body type.

For one example, the controller 180 may display a text" On your bottom half, you should wear clothes that make your hips look broader." in the square box (5) area as a description of the inverted triangular body type. For another example, the controller 180 may display ae text "You should create the illusion of a waist." in the square box (5) area as a description of the straight body type. For another example, the controller 180 may display a text "You should balance your top with your bottom half by wearing clothes that make your shoulders look broker." in the square box (5) area. For further example, the controller 180 may display a text "You should wear shaped and fitted lines that follow your body line." in the square box (5) area as a description of the hourglass body type.

Referring back to FIG. 4, if the body size report of S417 is completed, the controller 180 creates a virtual avatar based on the gender, the body size, and the face [S418], displays a look book screen (virtual fitting screen) including the virtual avatar wearing the found at least one fashion item [S419], and performs a cost payment operation for purchasing the at least one fashion item worn on the virtual avatar through authentication of the subject 200.[S420].

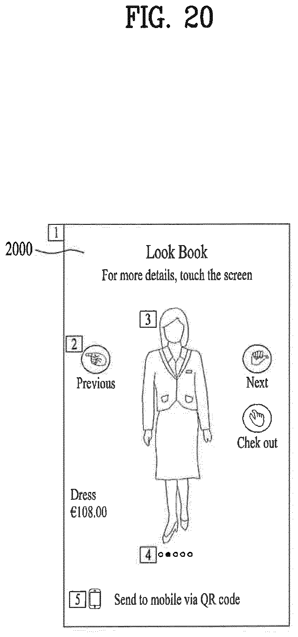

FIG. 20 is a diagram illustrating a look book screen including a virtual avatar in a simple fitting mode according to the present disclosure.

Referring to FIG. 20, a square box (1) area of a look book screen 2000 is an area in which a screen including a virtual avatar wearing at least one fashion item preferred by the subject 200 and a price information (e.g., dress 108.00 euros) for the at least one fashion item are displayed..

A square box (2) area of the look book screen 2000 is an area in which hand gestures guiding inputs of a hand gesture for moving a current page of the look book screen 2000 to a previous page, a hand gesture for moving to a next page, and a hand gesture for online pay (check out) of at least one fashion item worn on the virtual avatar are displayed.

A square box (3) area of the look book screen 2000 is an area in which the virtual avatar wearing the at least one fashion item is displayed.

A square box (4) area of the look book screen 2000 is an area in which page indicators indicating pages of virtual avatars wearing two or more fashion items respectively are displayed.

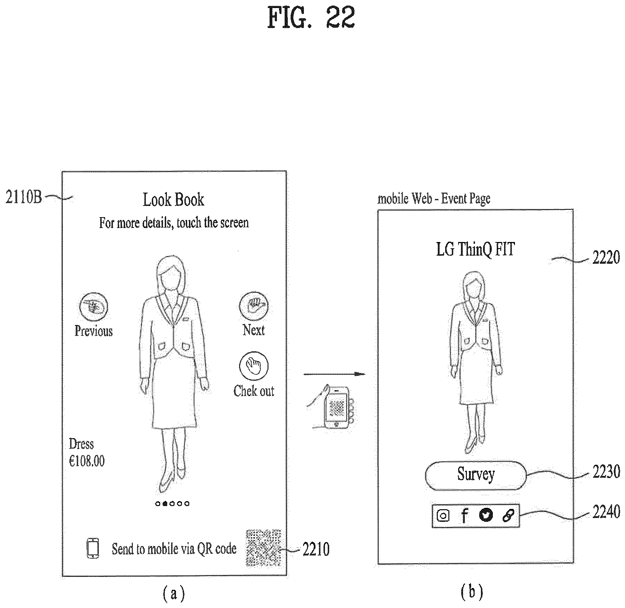

A square box (5) area of the look book screen 2000 is an area in which link information for transmitting information on the virtual avatar wearing the at least one fashion item to a mobile terminal of the subject 200 is displayed. For example, the link information may include a QR code. In this case, a process of transmitting the information on the virtual avatar to the mobile terminal of the subject 200 is performed identically in each of the simple fitting mode and the detailed fitting mode, and will be described in detail below with reference to FIG. 22.

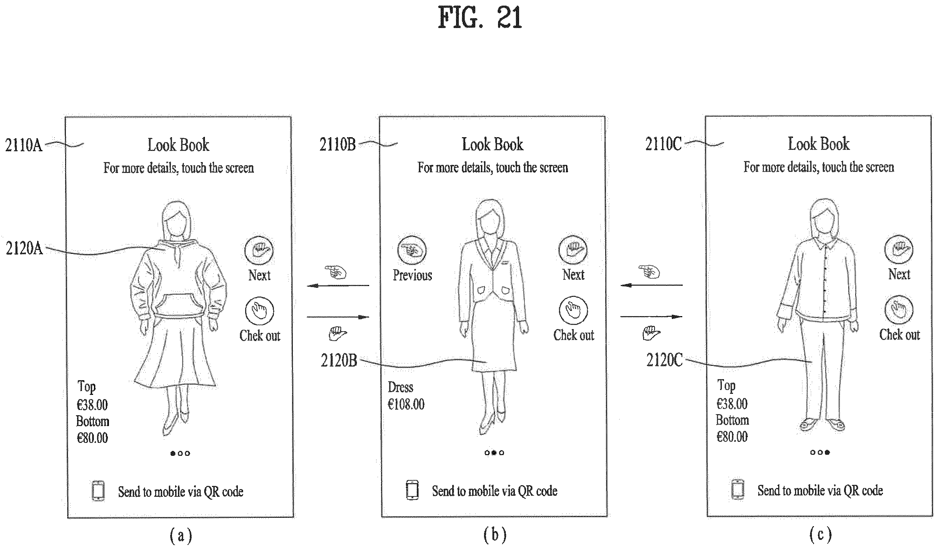

FIG. 21 is a diagram illustrating pages of virtual avatars wearing two or more fashion items in a simple fitting mode according to the present disclosure.

FIG. 21 (a) shows a first page 2110A of a look book screen on which a first virtual avatar 2120A wearing at least one first fashion item is displayed as a first recommended look. FIG. 21 (b) shows a second page 2110B of the look book screen on which a second virtual avatar 2120B wearing at least one second fashion item is displayed as a second recommended look. FIG. 21 (c) shows a third page 2110C of the look book screen on which a third virtual avatar 2120C wearing at least one third fashion item is displayed as a third recommended look. In this case, the first to third pages 2110A, 2110B and 2110C can move to one another in response to hand gestures of the subject 200.

FIG. 22 is a diagram illustrating a process for transmitting information on a virtual avatar in a look book screen to an external mobile terminal in a simple/detailed fitting mode according to the present disclosure.

As shown in FIG. 22 (a), the controller 180 displays a QR code 2210 for transmitting information on a virtual avatar wearing at least one fashion item included in the look book screen 2110B to the mobile terminal of the subject 200.

When the QR code is recognized through a QR code recognition function, a mobile terminal of the subject 200 displays a mobile web look book screen 2220 including the virtual avatar wearing the at least one fashion item, and may display an execution icon 2230 of an evaluation application for evaluation of the virtual avatar by external persons and an execution icon 2240 of at least one social network application for sharing the virtual avatar on a Social Network Service (SNS) in the mobile web look book screen 2220.

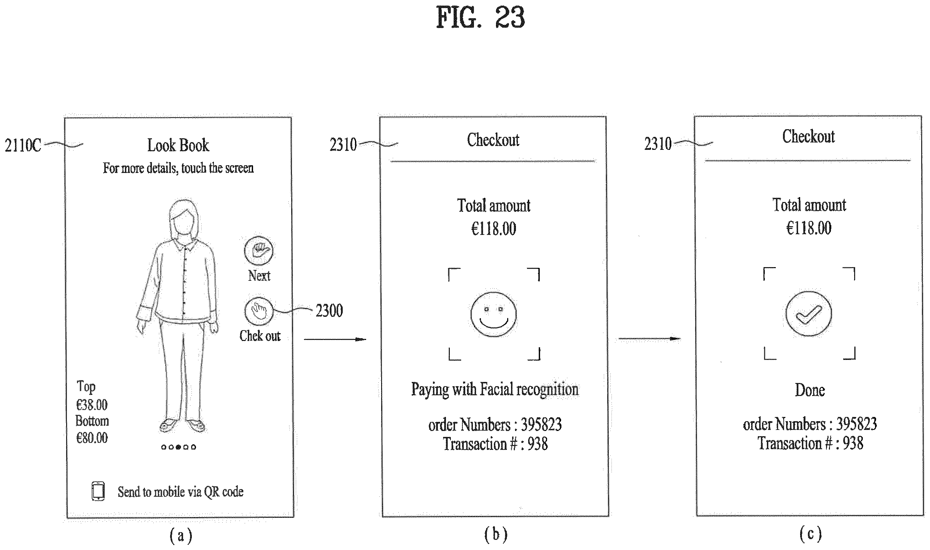

FIG. 23 is a diagram illustrating a process for making a payment for a fashion item worn on a virtual avatar in a simple fitting mode according to the present disclosure.

Referring to FIG. 23, the subject 200 may make a payment for a fashion item worn on the virtual avatar through face authentication of the subject 200.

When a payment menu 2300 for at least one fashion item worn on the virtual avatar in the look book screen 2110C is selected by a hand gesture of the subject 200 [FIG. 23 (a)], the controller 180 displays a payment authentication screen 2310 for the at least one fashion item and activates the camera 121 for face authentication of the subject 200 [FIG. 23 (b)].

When a face of the subject 200 in an image received through the camera 121 matches the face scanned in the step S416 of FIG. 4, as shown in FIG. 23 (c), the controller 180 performs a payment operation on the at least one fashion item using payment information of the subject 200 preset in the memory 170.

In this case, the payment information may include at least one of credit card information, prepaid card information, point card information, and bank account information of the subject 200.

Hereinafter, a detailed fitting mode of the present invention will be described in detail. In the following description of the detailed fitting mode, the same operations as the simple fitting mode described above may be omitted.

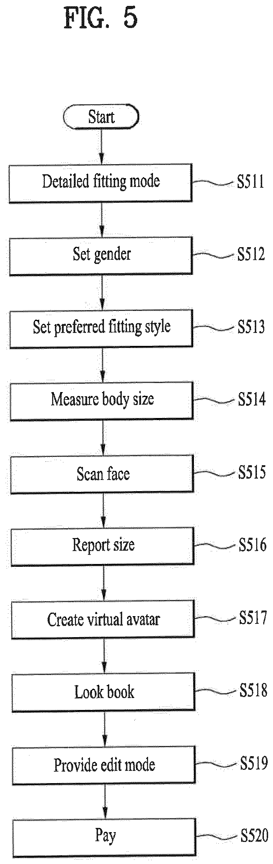

FIG. 5 is a flowchart illustrating an operating process of a detailed fitting mode of a virtual fitting service according to the present disclosure.

Referring to FIG. 5, if the detailed fitting mode is activated by the process of FIG. 3 [S511], the controller 180 displays a setting screen for receiving a gender setting from the subject 200 and receives a gender setting of the subject 200 from the subject 200 through the setting screen [S512].

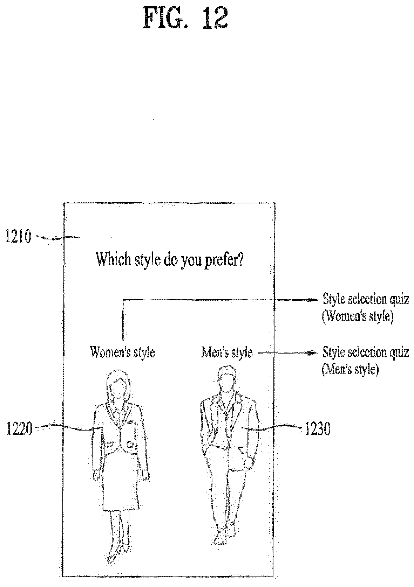

FIG. 12 is a diagram to describe a process for setting a gender of a subject in a detailed fitting mode according to the present disclosure.

Regarding the step S512 with reference to FIG. 12, in the detailed fitting mode, since all screen manipulations are performed by touch gestures of the subject 200, the controller 180 displays a female item 1220 indicating a female gender and a male item 1230 indicating a male gender in the setting screen 1210.

In addition, when the female item 1220 is selected, the controller 180 recognizes that the gender of the subject 200 is set as a female. When the male item 1230 is selected, the controller 180 recognizes that the gender of the subject 200 is set as a male.

Referring back to FIG. 5, once the gender of the subject 200 is set, the controller 180 displays a setting screen for receiving a setting of at least one preferred fitting style according to the gender set by the subject 200 and receives a setting of at least one preferred fitting style from the subject 200 through the setting screen [S513].

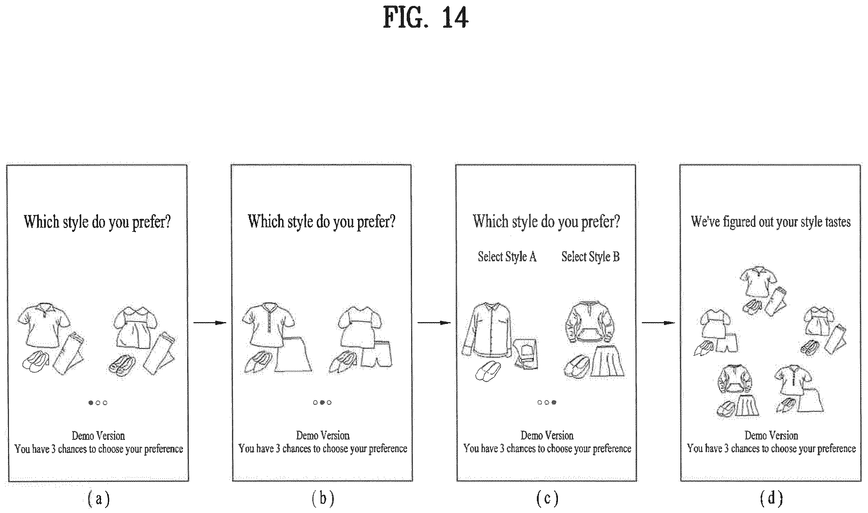

FIG. 14 is a diagram to describe a process for setting a preferred fitting style in a detailed fitting mode according to the present disclosure..

FIGs. 15 (a) to 15 (c) show pages of a screen including a multitude of preferred fitting styles for setting at least one preferred fitting style of the subject 200, respectively, and the subject 200 may set at least one preferred fitting style desired in each page by touching a preferred fitting style in each page.

In addition, as illustrated in FIG. 15 (d), the controller 180 may search and display at least one fashion item related to the at least one preferred fitting style set in FIGs. 14 (a) to 14 (c).

Referring back to FIG. 5, the controller 180 measures a body size of the subject 200 [S514] and scans a face of the subject 200 through the 3D camera [S515]

Since the body size measurement and face scanning processes of the steps S514 and S515 are the same as the above-described body size measurement and face scanning processes in the simple fitting mode, a detailed description thereof will be omitted.

Next, if the body size measurement and face scanning of the subject 200 are completed, the controller 180 displays a report result screen for the measured body size of the subject 200 [S516].

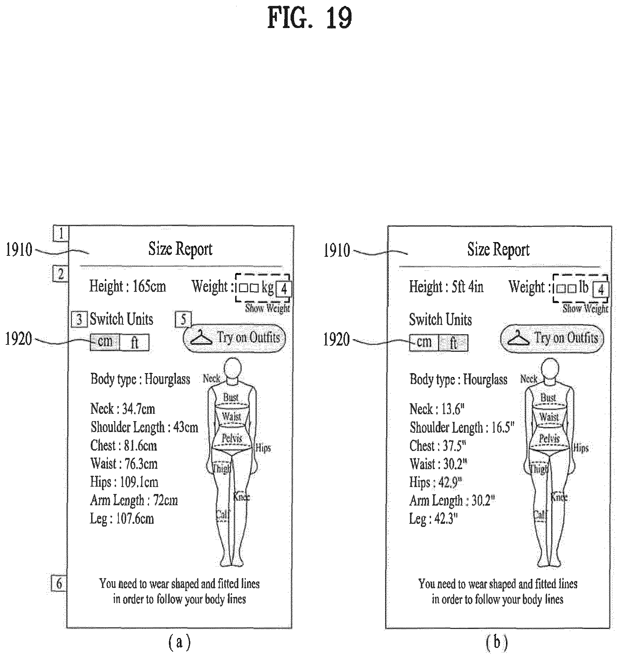

FIG. 19 is a diagram illustrating a subject's body size report screen provided in a detailed fitting mode according to the present disclosure.

Referring to FIG. 19, a body size report screen 1910 may represent the total height, weight, shoulder width, neck circumference, shoulder, chest, waist, hip, arm length, and leg length of the subject 200.

In this case, the subject 200 may select a unit change menu 1920 in the body size report screen 1910, thereby changing the unit for the body sizes.

For example, in FIG. 19(a), the body sizes of the subject 200 are shown in units of centimeters (cm) and kilograms (kg). In FIG. 19 (b), the body sizes of the subject 200 are shown in units of inches and pounds (lbs). The controller 180 may switch the centimeter (cm) and kilogram (kg) units to inch and pound (lb) units whenever a unit switch menu 1820 is selected from the subject 200.

Meanwhile, in FIG. 19, a square box (1) area indicates an area in which a title of the body size report screen is displayed. A square box (2) area indicates an area in which a height of the subject is displayed. A square box (3) area indicates an area in which the unit change menu 1820 and the body sizes of the subject 200 are displayed. A square box (4) area indicates an area in which weight of the subject 200 is displayed, but is not displayed until it is touched by the subject 200. A square box (5) area indicates an area in which a shortcut menu for a direct access to a look book screen including a virtual avatar wearing at least one fashion item. A square box (6) area indicates an area in which a description of body type definition based on the measured body sizes and gender is displayed. Since the description of the body type is the same as described in FIG. 18, a detailed description thereof will be omitted.

Referring back to FIG. 5, if the body size report of S516 is completed, the controller 180 creates a virtual avatar based on the gender, the body size, and the face [S517], displays a look book screen (virtual fitting screen) including the virtual avatar wearing the found at least one fashion item [S518], provides an edit mode for the look book screen [S519], and performs a cost payment operation for purchasing the at least one fashion item worn on the virtual avatar through authentication of the subject 200.[S520].

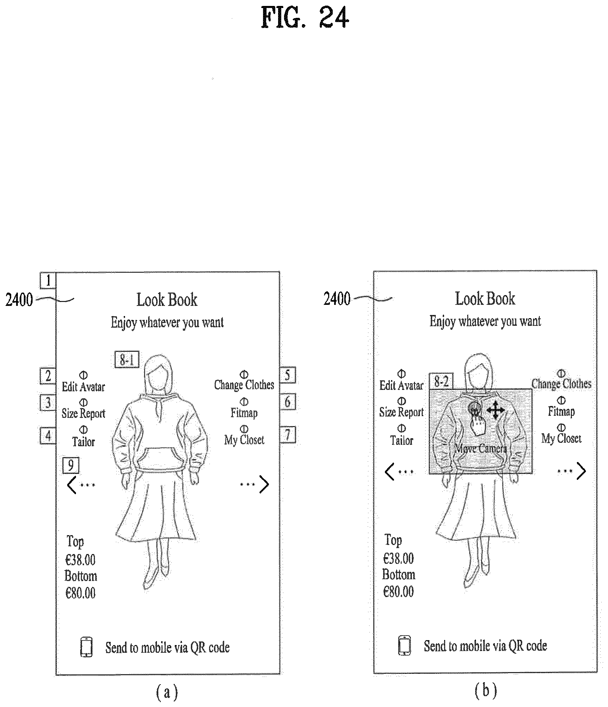

FIG. 24 is a diagram illustrating a look book screen including a virtual avatar in a detailed fitting mode according to the present disclosure.

Referring to FIG. 24 (a) and FIG. 24 (b), a square box (1) area of the look book screen 2000 is an area in which a screen including a virtual avatar wearing at least one fashion item preferred by the subject 200 and price information (e.g., Top 38.00 euros and Bottom 80.00 euros) is displayed.

A square box (2) area of a look book screen 2400 is an area in which a virtual avatar edit menu is displayed as a sort of the edit mode provided in the step S519.

A square box (3) area of the look book screen 2400 is an area in which a size report menu for direct access to a previously displayed body size report screen is displayed as a sort of the edit mode provided in the step S519.

A square box (4) area of the look book screen 2400 is an area in which a tailor menu for adjusting a length of a bottom of at least one fashion item worn on a virtual avatar is displayed as a sort of the edit mode provided in the step S519.

A square box (5) area of the look book screen 2400 is an area in which a change menu for changing at least one of at least one fashion item currently worn on a virtual avatar to another fashion item is displayed, as a sort of the edit mode provided in the step S519.

A square box (6) area of the look book screen 2400 is an area in which a fit map menu for recommending a size for a fashion item of a corresponding body part worn on a virtual avatar is displayed based on an actual body size of a user, as a sort of the edit mode provided in the step S519.

A square box 7 area of the look book screen 2400 is an area in which a My Closet menu for enabling a current virtual avatar to try on pre-stored fashion items is displayed as a sort of the edit mode provided in the step S519.

A square box (8-1) area of the look book screen 2400 is an area in which the virtual avatar is displayed.

A square box (8-2) area of the look book screen 2400 is an area in which a touch gesture guide for manipulating the virtual avatar or at least one fashion item worn on the virtual avatar is displayed.

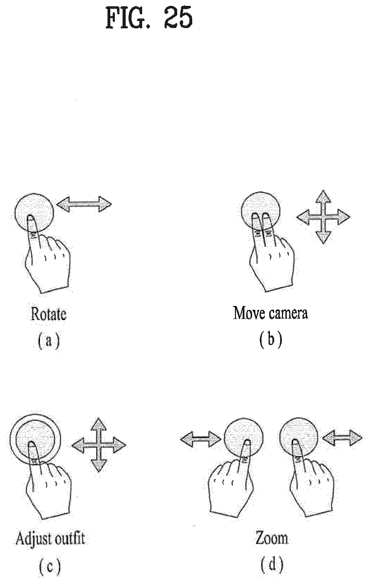

FIG. 25 is a diagram illustrating a process for controlling a look book screen via a touch gesture in a detailed fitting mode according to the present disclosure.

First of all, as shown in FIG. 25 (a), when a portion of an virtual avatar wearing at least one fashion item is touched by the subject 200 and dragged in a horizontal direction, the controller 180 may rotate the virtual avatar in the horizontal direction centering around the center of the touched portion in response to the drag in the horizontal direction.

In addition, as shown in FIG. 25 (b), when two fingers of the subject 200 are dragged upward, downward, leftward, or rightward while being touched on the virtual avatar, the controller 180 may move the camera 121 in the dragged direction. That is, the camera 121 according to the present disclosure has a structure capable of moving upward, downward, leftward, and rightward, and the controller 180 may move the camera 121 in a direction corresponding to a touch direction of the subject 200.

In addition, as shown in FIG. 25 (c), when a portion of the virtual avatar is dragged in any one of an upward direction, a downward direction, a left direction, and a right direction while being long-touched by the subject 200, the controller 180 may adjust a size or length of an outfit for the portion.

In addition, as shown in FIG. 25 (d), when first and second portions of the virtual avatar are dragged in opposite directions while being touched by the subject 200, the controller 180 may enlarge and display one portion of the virtual avatar corresponding between the first and second portions.

Meanwhile, when the change menu displayed in the square box (5) area of the look book screen 2400 is touched by the subject 200, the controller 180 may provide a fashion item change screen capable of changing at least one of at least one or more fashion items currently worn on the virtual avatar into another fashion item. Subsequently, the subject 200 may select desired fashion items through the fashion item change screen, enable the virtual avatar to try on them, and then purchase them.

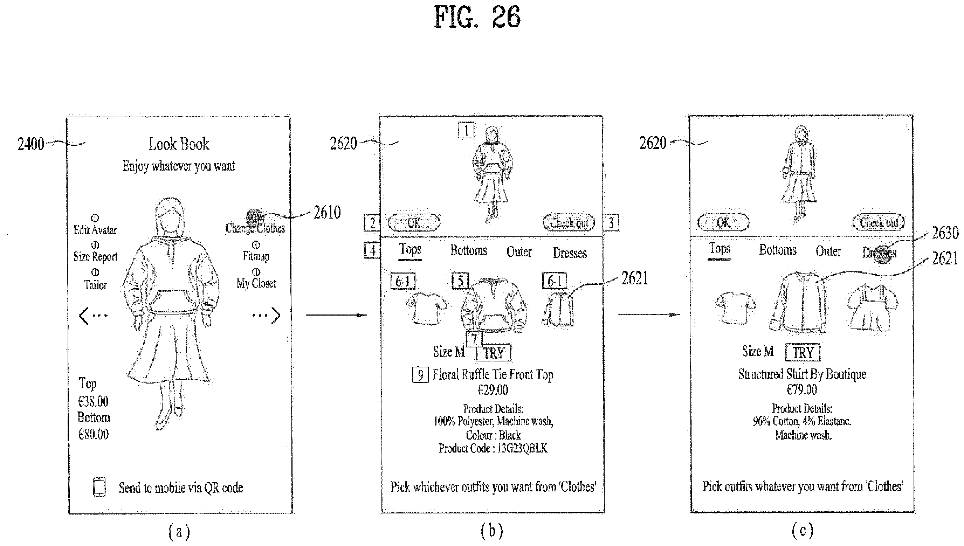

FIGs. 26 to 29 are diagrams illustrating a process for changing and purchasing a fashion item worn on a virtual avatar in a detailed fitting mode according to the present disclosure.

First of all, when a change menu 2610 is touched in the look book screen 2400 [FIG. 26 (a)], the controller 180 displays a fashion item change screen 2620 [FIG. 26(b)].

In this case, as shown in FIG. 26 (b), a square box (1) area in the fashion item change screen 2620 is a preview area in which at least one fashion item selected through the fashion item change screen 2620 is shown by being worn on a virtual avatar. In this case, the virtual avatar displayed in the preview area is displayed in the form of a 3D virtual avatar that can be rotated 360 degrees in response to a touch drag of the subject 200.

In addition, a square box (2) area in the fashion item change screen 2620 is an area in which an OK button for displaying a look book screen of the virtual avatar wearing the fashion item changed through the fashion item change screen 2620 is displayed.

In addition, a square box (3) area in the fashion item change screen 2620 is an area in which a pay button for payment of a fashion item finally changed for the virtual avatar through the fashion item change screen 2620 is displayed.

In addition, a square box (4) area in the fashion item change screen 2620 is an area in which category tabs of fashion items are displayed.

In addition, a square box (5) area in the fashion item change screen 2620 is an area in which a fashion item currently worn on a virtual avatar in the preview area among fashion items belonging to a category selected from the category tabs is displayed.

In addition, a square box (6-1) area in the fashion item change screen 2620 is an area in which fashion items in a previous or next order of a fashion item worn on the virtual avatar in the preview area is displayed.

In addition, a square box (7) area in the fashion item change screen 2620 is an area in which a wearing button for enabling a virtual avatar to try on a selected item is displayed.

In addition, a square box (9) area in the fashion item change screen 2620 is an area in which information (including at least one of title, price, size, material, color, and code number) on a fashion item worn on a virtual avatar in the preview area is displayed.

As shown in FIG. 26 (b), when the top category tab (Tops) in the square box (4) area is touched, the controller 180 searches for top clothes and displays the top clothes in the square box (5) and (6-1) areas. If a shirt 2621among the top clothes is touched, as shown in FIG. 26 (c), the controller 180 displays the virtual avatar wearing the shirt 2621 in a preview form.

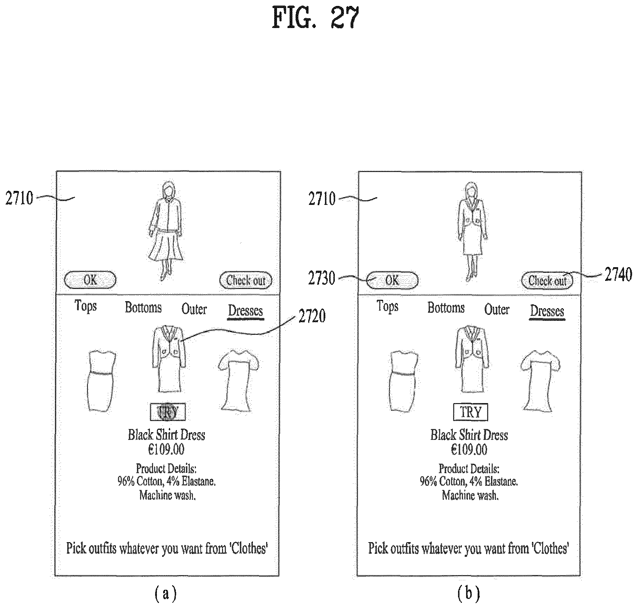

In addition, as shown in FIG. 26 (c), when a dress category tab (Dresses) 2630 in the square box (4) area is touched, as shown in FIG. 27 (a), the controller 180 searches for dresses and displays them in the fashion item change screen 2710. If a specific dress 2720 is touched among the dresses, as shown in FIG. 27 (b), the controller displays the virtual avatar wearing the dress 2720 in a preview form.

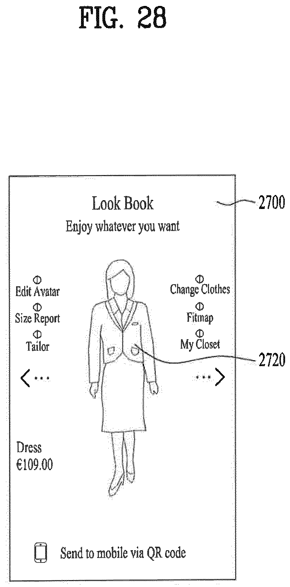

Meanwhile, as shown in FIG. 27 (b), when an OK button 2730 is touched by the subject 200 while the virtual avatar wearing the dress 2720 is displayed in the preview form, as shown in FIG. 28, the controller 180 displays a look book screen 2700 of the virtual avatar wearing the dress 2720 changed through the fashion item change screen 2620.

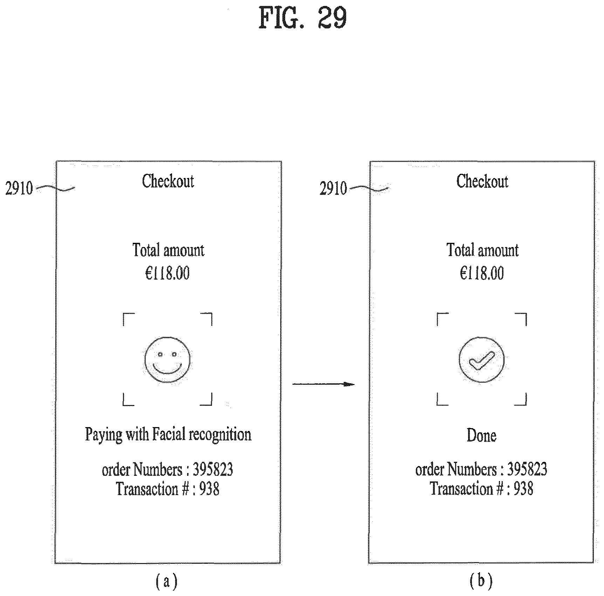

Meanwhile, as shown in FIG. 27 (b), when a pay button 2740 is touched by the subject 200 while the virtual avatar wearing the dress 2720 is displayed in the preview form, as shown in FIG. 29 (a), the controller 180 displays a payment authentication screen 2910 for the dress 2720 and activates the camera 121 for the face authentication of the subject 200.

When a face of the subject 200 in the image received through the camera 121 matches the face scanned in the step S515 of FIG. 5, as shown in FIG. 29 (b), the controller 180 performs a payment operation on the dress 2720 using payment information of the subject 200 preset in the memory 170. In this case, the payment information may include at least one of credit card information, prepaid card information, point card information, and bank account information of the subject 200.

Meanwhile, when the virtual avatar edit menu displayed in the square box (2) area of the look book screen 2400 is touched by the subject 200, the controller 180 may provide a virtual avatar edit screen capable of body type change, hair style change and glasses wearing of a current virtual avatar and the subject 200 may edit the virtual avatar in a desired style through the virtual avatar edit screen..

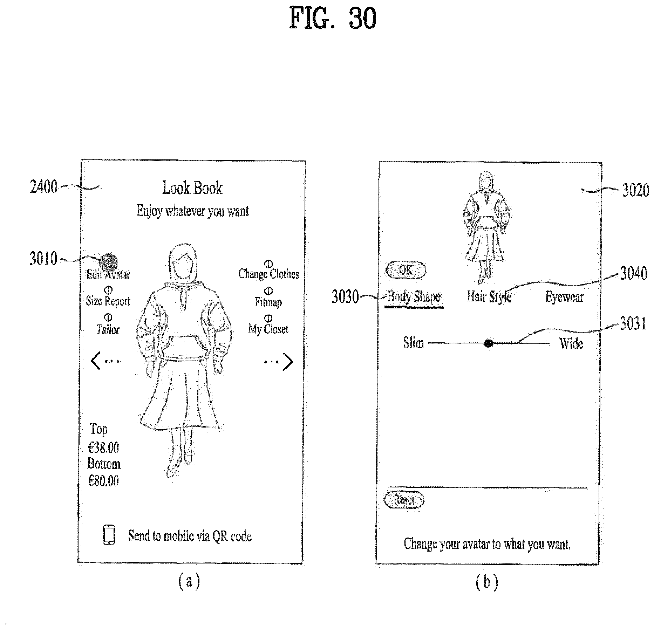

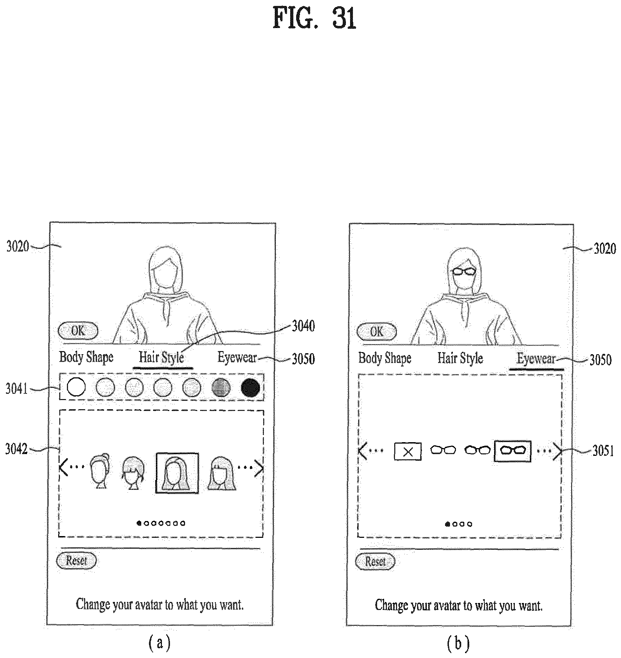

FIG. 30 and FIG. 31 are diagrams illustrating a process for editing a virtual avatar in a detailed fitting mode according to the present disclosure.

First of all, when the avatar edit menu 3010 is touched in the look book screen 2400 [FIG. 30 (a)], the controller 180 displays a virtual avatar edit screen 3020 [FIG. 30(b)].

In this case, as shown in FIG. 30 (b), a preview area located at the top, on which a virtual avatar applied to the editing result is displayed, is included in the virtual avatar edit screen 3020.

In addition, virtual avatar edit tabs including a body type change tab, a hair style change tab, and a glasses wearing tab are included in the virtual avatar edit screen 3020.

For example, when the virtual avatar edit screen 3020 is initially displayed, the body type change tab 3030 among the virtual avatar edit tabs is selected and displayed as a default.

When the body type change tab 3030 is selected and displayed, the controller 180 may display a body type change UI 3031 for body type change of the virtual avatar. If a weight-gaining degree for a body type of the virtual avatar is set through the body type change UI 3031, the controller 180 may control a body type corresponding to the set weight-gaining degree to be displayed in the preview area by being reflected by the virtual avatar. For example, the subject 200 may set the weight-gaining degree for the virtual avatar by dragging the body type change UI 3031 left and right.

Meanwhile, when the body type change tab 3030 is selected and displayed and the virtual avatar cannot be displayed in a full size in the preview area due to a predetermined size of the preview area, the controller 180 may display the virtual avatar by reducing the virtual avatar to a size displayable in full size in the preview area, whereby the subject 200 may check the entire body type of the virtual avatar.

Next, as shown in FIG. 30 (b), if the style change tab 3040 among the virtual avatar edit tabs is touched, as show in FIG. 31 (a), the controller displays a color change UI 3041 and a hair style change UI 3042 for a hair style and color change of the virtual avatar and may control a color and hair style, which are touched and selected via the color change UI 3041 and the hair style change UI 3042, to be displayed in the preview area by being reflected by the virtual avatar.

In doing so, if the selected color and hair style are reflected by the virtual avatar in the preview area, the controller 180 may enlarge and display the virtual avatar in a preset size to facilitate the subject to check the color and hair style reflected by the virtual avatar.

Next, as shown in FIG. 31 (a), if the glasses wearing tab 3050 is touched among the virtual avatar editing tabs, as shown in FIG. 31 (b), the controller 180 may display a glasses UI 3051 for wearing glasses on the virtual avatar and control glasses, which are selected by being touched through the glasses UI 3051, to be displayed in the preview area in a manner of being worn on the virtual avatar.

In this case, when the selected glasses are worn on the virtual avatar in the preview area, the controller 180 may enlarge the virtual avatar to a preset size so that the subject 200 may easily check the glasses worn on the virtual avatar.

Meanwhile, when the fit map menu displayed in the square box (6) area of the look book screen 2400 is touched by the subject 200, the controller 180 provides a fit map screen recommending the size of a fashion item currently worn on a body part by the virtual avatar based on a user's actual body size and the subject 200 may know an optimal wearing size of a fashion item based on its own body size through the fit map screen. Hereinafter, the function of recommending the size of a fashion item currently worn on a body part by the virtual avatar based on a user's actual body size will be described by being referred to as a fit map recommendation function.

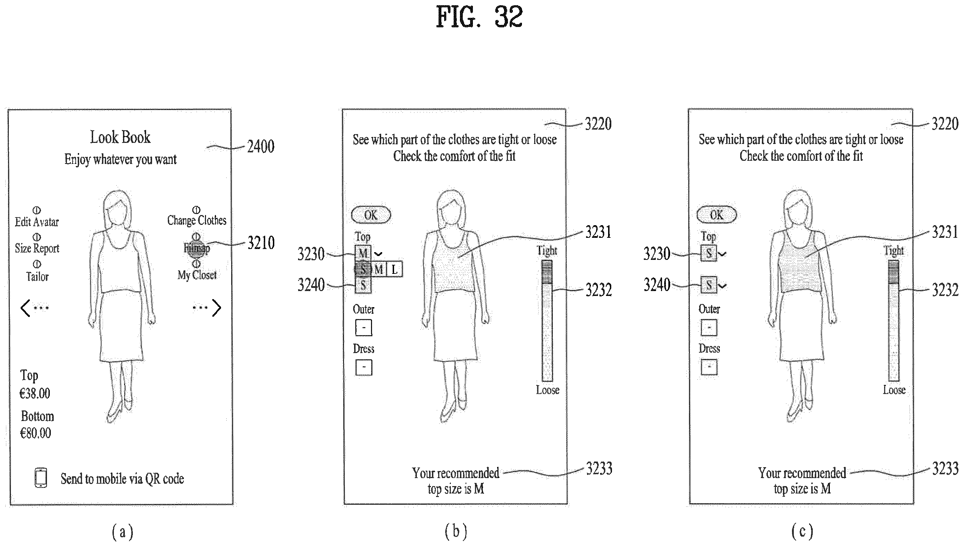

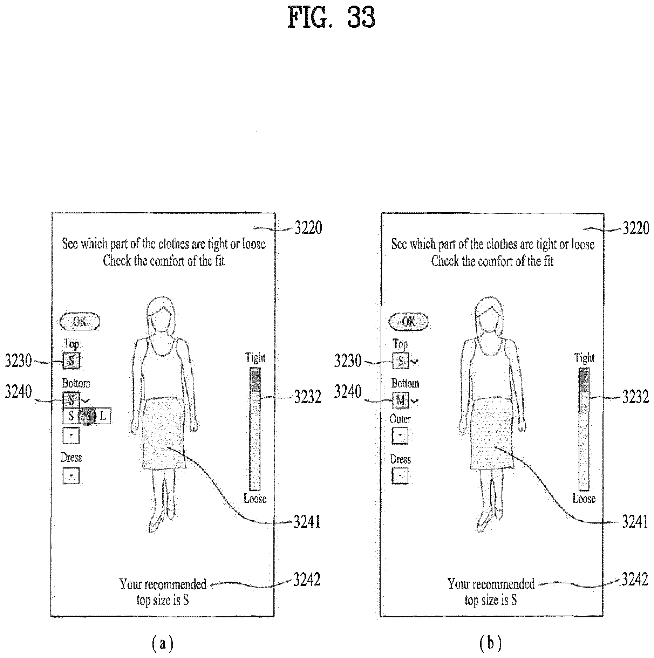

FIG. 32 and FIG. 33 are diagrams illustrating a process for providing a fit map function in a detailed fitting mode according to the present disclosure.

First of all, if the fit map menu 3210 is touched in the look book screen 2400 [FIG. 32 (a)], the controller 180 displays a fit map screen 3220 [FIG. 32 (b)].

In this case, as shown in FIG. 32 (b), a preview area in which a virtual avatar applied to the editing result is displayed is displayed in the center of the fit map screen 3220.

In addition, a size change area including at least one of a top size change UI 3230, a bottom size change UI 3240, an outer size change UI, and a dress size change UI is displayed in the left area of the fit map screen 3220.

In addition, an indicator 3232 indicating a level of tightness or looseness of clothes selected by the subject 200 among clothes worn on the virtual avatar in a multitude of colors is displayed in the right area of the fit map screen 3220.

In addition, information 3233 indicating a recommended size of clothes selected by the subject 200 among clothes worn on the virtual avatar is displayed in the lower area of the fit map screen 3220.

Namely, when the subject 200 requests a size change of the clothes through an UI of desired clothes among UIs in the size change area, the controller 180 may determine one of tightness, fit, or looseness for the size of the change-requested clothes size based on the actual body size of the subject 200 and display the information 3233 indicating the determination result.

For example, as shown in FIG. 32 (b), when a top 3231 of the clothes worn on the virtual avatar is touched and selected, the controller 180 determines one of tightness, fit, or looseness for the size of the top 3231 currently worn on the virtual avatar based on at least one of shoulder width, neck circumference, chest circumference, waist circumference, and arm length included in an upper body size among pre-measured body sizes of the subject 200.

For example, when the current size of the top 3231 worn on the virtual avatar is a medium size (M), if the medium size of the top 3231 is determined as fit based on the upper body size of the subject 200, the controller 180 displays the color of the top 3231 as a color indicating the 'fit' among the colors in the indicator 3232. In addition, the controller 180 displays information 3233 indicating that the medium size of the top 3231 is fit.

Meanwhile, when a request for changing the medium size (M) of the top worn on the virtual avatar into a small size (S) is made by the subject 200 through a top size change UI 3230, the controller 180 determines one of tightness, fit, or looseness for the change requested clothes size based on at least one of shoulder width, neck circumference, chest circumference, waist circumference, and arm length included in the upper body size among the pre-measured body sizes of the subject 200.

If determining tightness, as shown in FIG. 32 (c), the controller 180 displays the color of the top 3231 as a color indicating the tightness among the colors in the indicator 3232. In addition, the controller 180 displays the information 3233 indicating that the medium size of the top 3231 is fit.

For another example, as shown in FIG. 33 (a), when a bottom 3241 of the clothes worn on the virtual avatar is touched and selected, the controller 180 determines a size of the bottom 3241 currently worn on the virtual avatar as one of tightness, fit or looseness based on at least one of waist, hip, and leg lengths included in the lower body size among the pre-measured body sizes of the subject 200.

For example, when the current size of the bottom 3241 worn on the virtual avatar is a small size (S), if the small size of the bottom 3241 is determined as fit based on the lower body size of the subject 200, the controller 180 displays the color of the bottom 3241 as a color indicating the 'fit' among the colors in the indicator 3232. In addition, the controller 180 displays information 3242 indicating that the small size of the bottom 3241 is fit.

Meanwhile, when a request for changing the small size (S) of the bottom 3241 worn on the virtual avatar into a medium size (M) is made by the subject 200 through a bottom size change UI 3240, the controller 180 determines one of tightness, fit, or looseness for the change requested size of the bottom 3241 based on the waist, hip, and leg lengths included in the lower body size among the pre-measured body sizes of the subject 200.

If determining the looseness, as shown in FIG. 33 (b), the controller 180 displays the color of the bottom 3241 as a color indicating the looseness among the colors in the indicator 3232. In addition, the controller 180 displays the information 3242 indicating that the small size of the bottom 3241 is fit.

Meanwhile, when a My Closet menu displayed in the square box (7) area of the look book screen 2400 is touched by the subject 200, the controller 180 displays a My Closet screen for enabling a current virtual avatar to directly wear pre-stored fashion items and the subject 200 may facilitate the current virtual avatar to wear the pre-stored fashion items.

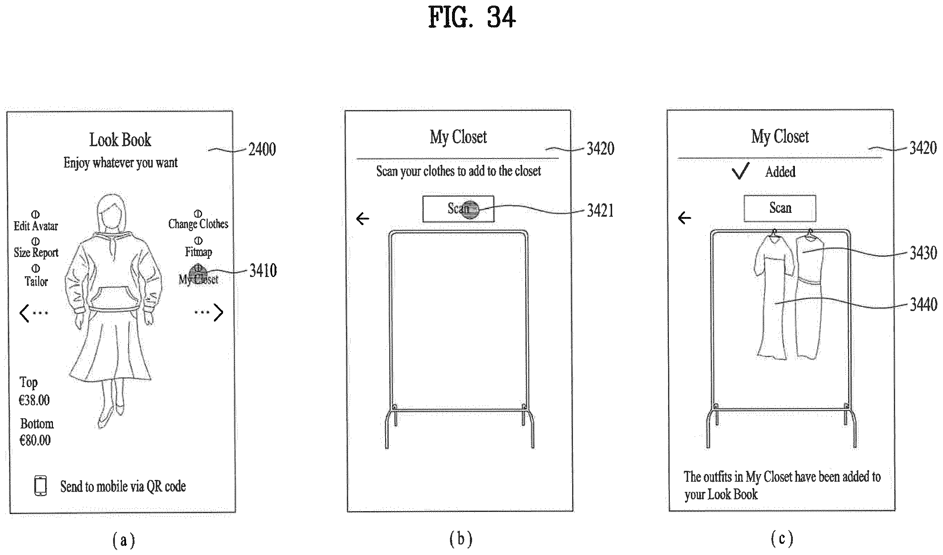

FIG. 34 is a diagram illustrating a process for providing a My Closet function in a detailed fitting mode according to the present disclosure.

If the My Closet menu 3410 is touched in the look book screen 2400 [FIG. 34 (a)], the controller 180 displays the My Closet screen 3420 [FIG. 34 (b)].

In this case, when a scan button 3421 for scanning at least one pre-stored fashion item is included in the My Closet screen 3420, if the scan button 3421 is touched by the subject 200, as shown in FIG. 34 (c), the controller 180 scans and displays at least one or more fashion items 3430 and 3440 pre-stored in the memory 170 and then displays the virtual avatar wearing the scanned at least one or more fashion items 3430 and 3440.

Meanwhile, when the tailor menu displayed in the square box (4) area of the look book screen 2400 is touched by the subject 200, the controller 180 displays a tailor screen for changing a type of shoes currently worn by a virtual avatar and adjusting a length of a bottom currently worn by the virtual avatar and the subject 200 may easily change the type of the shoes currently worn by the virtual avatar and easily adjust the length of the bottom to be suitable for the changed type of the shoes.

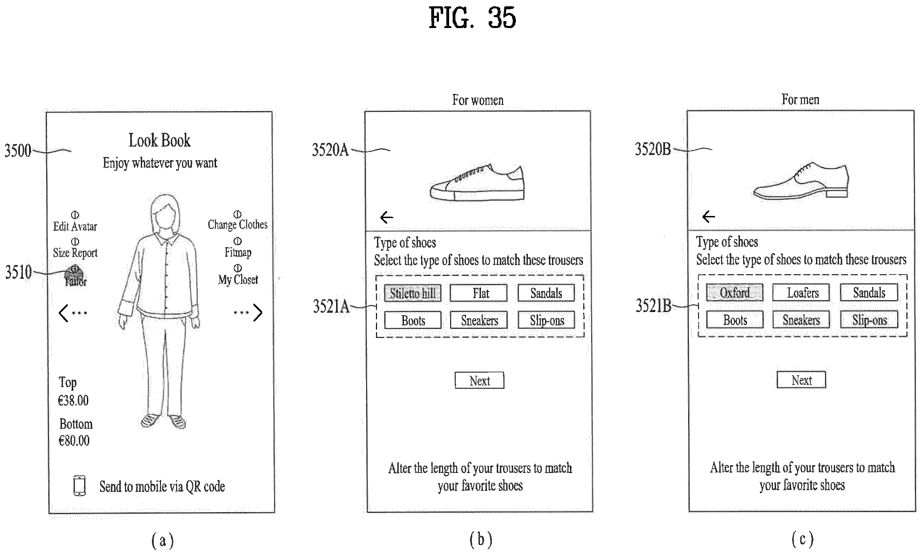

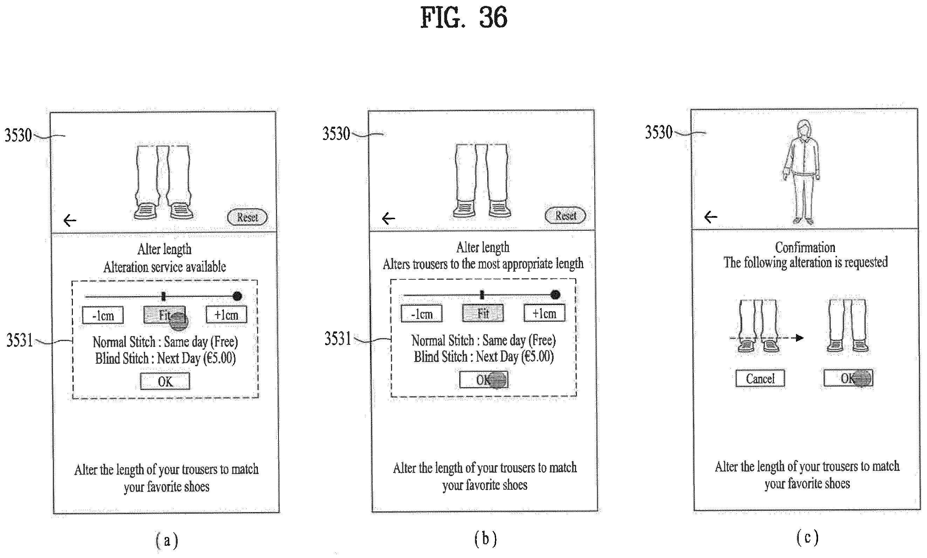

FIG. 35 and FIG. 36 are diagrams illustrating a process for providing a tailor function in a detailed fitting mode according to the present disclosure.

First of all, as shown in FIG. 35 (a), when the tailor menu 3510 is touched in the look book screen 3500, as shown in FIG. 35 (b) and FIG. 35 (c), the controller 180 displays a women's shoes type change screen 3520A or a men's shoes type change screen 3520B according to the gender of the virtual avatar.

In this case, a first change UI 3521A for changing the type of women's shoes is displayed in the women's shoes type change screen 3520A, and a second change UI 3521B for changing the type of men's shoes is displayed in the men's shoes type change screen 3520B.

When a specific women's shoe is touched and selected by the subject 200 through the first change UI 3521A or a specific men's shoe is touched and selected by the subject 200 through the second change UI 3521B, as shown in FIG. 36 (a) and FIG. 36 (b), the controller 180 displays a bottom length change screen 350 for changing the length of bottoms.

If the length to be changed is touched and set through a third change UI 3531 for changing the length of the bottoms in the bottom length change screen 350, as shown in FIG. 36C, the controller 180 displays the virtual avatar reflecting the changed shoes and the length-changed bottoms.

The above detailed description of the present disclosure should not be construed as being limitative in all terms, but should be considered as being illustrative. The scope of the present disclosure should be determined by reasonable analysis of the accompanying claims, and all changes in the equivalent range of the present disclosure are included in the scope of the present disclosure.

## Claims

1. An apparatus for providing a virtual fitting, the apparatus comprising:
a touchscreen;
a camera receiving an image of a subject in front; and
a controller configured to activate a virtual fitting mode if detecting an input of a hand gesture of the subject or an input of a touch gesture of the subject on the touchscreen, inquire a gender of the subject and at least one preferred style, search for at least one fashion item related to the inquired gender and the preferred style, the fashion item including at least one of at least one clothes, shoes and accessory, measure a body size of the subject, scan a face of the subject, create a virtual avatar based on the gender, the body size and the face, and display a virtual fitting screen including the avatar wearing the found fashion item on the touchscreen.

2. The apparatus of claim 1, wherein the controller activates a hand gesture based virtual fitting mode based on recognizing a preset hand gesture of the subject through the image and displays information for guiding two or more hand gestures for manipulation of the virtual fitting screen on the virtual fitting screen.

3. The apparatus of claim 3, wherein the controller measures the body size of the subject by making a request for two or more body postures to the subject.

4. The apparatus of claim 3, wherein the controller displays guide information for guiding the subject to follow the body postures on the virtual fitting screen.

5. The apparatus of claim 1, wherein the controller scans the face of the subject by requesting the subject to move a head in a specific pattern.

6. The apparatus of claim 5, wherein the controller displays guide information for guiding the subject to move the head in the specific pattern on the virtual fitting screen.

7. The apparatus of claim 1, wherein the controller displays price information of the fashion item worn on the virtual avatar in the virtual fitting screen.

8. The apparatus of claim 1, wherein the controller displays link information for transmitting information of the virtual avatar wearing the fashion item to a mobile terminal of the subject in the virtual fitting screen.

9. The apparatus of claim 1, wherein the controller performs a cost payment for the fashion item via face recognition of the subject.

10. The apparatus of claim 1, wherein the controller displays a User Interface (UI) for a size change of the fashion item worn on the virtual avatar in the virtual fitting screen and wherein based on the body size, the controller displays information indicating a recommended size for the fashion item in the virtual fitting screen.

11. A method for providing a virtual fitting in a virtual fitting providing device, the method comprising:
receiving an image of a subject in front through a camera;
activating a virtual fitting mode if detecting an input of a hand gesture of the subject or an input of a touch gesture of the subject on the touchscreen;
inquiring a gender of the subject and at least one preferred style;
searching for at least one fashion item related to the inquired gender and the preferred style, the fashion item including at least one of at least one clothes, shoes and accessory;
measuring a body size of the subject;
scanning a face of the subject;
creating a virtual avatar based on the gender, the body size and the face; and
displaying a virtual fitting screen including the avatar wearing the found fashion item on the touchscreen.

12. The method of claim 11, the activating the virtual fitting mode, comprising:
activating a hand gesture based virtual fitting mode based on recognizing a preset hand gesture of the subject through the image; and
displaying information for guiding two or more hand gestures for manipulation of the virtual fitting screen on the virtual fitting screen.

13. The method of claim 11, the measuring the body size, comprising measuring the body size of the subject by making a request for two or more body postures to the subject.

14. The method of claim 13, the measuring the body size, comprising displaying guide information for guiding the subject to follow the body postures on the virtual fitting screen.

15. The method of claim 11, the scanning the face, comprising scanning the face of the subject by requesting the subject to move a head in a specific pattern.

16. The method of claim 15, the scanning the face, comprising displaying guide information for guiding the subject to move the head in the specific pattern on the virtual fitting screen.

17. The method of claim 11, further comprising displaying price information of the fashion item worn on the virtual avatar in the virtual fitting screen.

18. The method of claim 11, further comprising displaying link information for transmitting information of the virtual avatar wearing the fashion item to a mobile terminal of the subject in the virtual fitting screen.

19. The method of claim 11, further comprising performing a cost payment for the fashion item via face recognition of the subject.

20. The method of claim 11, further comprising:
displaying a User Interface (UI) for a size change of the fashion item worn on the virtual avatar in the virtual fitting screen; and
based on the body size, displaying information indicating a recommended size for the fashion item in the virtual fitting screen.
